# EUROPEAN PATENT SPECIFICATION

(11) **EP 2 010 485 B1**
(45) Date of publication and mention of the grant of the patent: **17.10.2012**
(21) Application number: 07733612.1
(22) Date of filing: 11.04.2007
(51) Int. Cl.: C07D 205/04, C07D 401/12, C07D 401/14, C07D 405/14, A61K 31/435, A61K 31/495, A61P 3/00

(54) **AZETIDINE DERIVATIVES AS G-PROTEIN COUPLED RECEPTOR (GPR119 ) AGONISTS**
AZETIDINDERIVATE ALS AGONISTEN DES G-PROTEIN-GEKOPPELTEN REZEPTORS (GPR119)
DÉRIVÉS D'AZÉTIDINE UTILISÉS EN TANT QU'AGONISTES DU RÉCEPTEUR COUPLÉ À LA PROTÉINE G (GPR119)

(30) Priority: 11.04.2006 GB 0607196
(43) Date of publication of application: 07.01.2009
(73) Proprietor: PROSIDION LTD, Oxford, Oxfordshire OX4 6LT (GB)
(72) Inventor: FYFE, Matthew, Colin, Thor, Oxford Oxfordshire OX4 6LT (GB); GATTRELL, William, Oxford Oxfordshire OX4 6LT (GB); RASAMISON, Chrystelle, Marie, Oxford Oxfordshire OX4 6LT (GB)
(74) Representative: Rands, Peter David
(86) International application number: PCT/GB2007/050190
(87) International publication number: WO 2007/116230

(56) References cited:
- WO-A-03/077907
- WO-A-2005/061489
- ROLLAND ET AL.: "G-Protein-Coupled Receptor Affinity Prediction Based on the Use of a profiling Dataset: QSAR Design, Synthesis, and Experimental Validation" J. MED. CHEM., vol. 48, no. 21, 2005, pages 6563-6574, XP002439868

## Description

### BACKGROUND OF THE INVENTION

The present invention is directed to G-protein coupled receptor (GPCR) agonists. In particular, the present invention is directed to agonists of GPR119 that are useful for the treatment of obesity, e.g. as regulators of satiety, metabolic syndrome and for the treatment of diabetes.

Obesity is characterized by an excessive adipose tissue mass relative to body size. Clinically, body fat mass is estimated by the body mass index (BMI; weight(kg)/height(m)²), or waist circumference. Individuals are considered obese when the BMI is greater than 30 and there are established medical consequences of being overweight. It has been an accepted medical view for some time that an increased body weight, especially as a result of abdominal body fat, is associated with an increased risk for diabetes, hypertension, heart disease, and numerous other health complications, such as arthritis, stroke, gallbladder disease, muscular and respiratory problems, back pain and even certain cancers.

Pharmacological approaches to the treatment of obesity have been mainly concerned with reducing fat mass by altering the balance between energy intake and expenditure. Many studies have clearly established the link between adiposity and the brain circuitry involved in the regulation of energy homeostasis. Direct and indirect evidence suggest that serotonergic, dopaminergic, adrenergic, cholinergic, endocannabinoid, opioid, and histaminergic pathways in addition to many neuropeptide pathways (e.g. neuropeptide Y and melanocortins) are implicated in the central control of energy intake and expenditure. Hypothalamic centres are also able to sense peripheral hormones involved in the maintenance of body weight and degree of adiposity, such as insulin and leptin, and fat tissue derived peptides.

Drugs aimed at the pathophysiology associated with insulin dependent Type I diabetes and non-insulin dependent Type II diabetes have many potential side effects and do not adequately address the dyslipidaemia and hyperglycaemia in a high proportion of patients. Treatment is often focused at individual patient needs using diet, exercise, hypoglycaemic agents and insulin, but there is a continuing need for novel antidiabetic agents, particularly ones that may be better tolerated with fewer adverse effects.

Similarly, metabolic syndrome (syndrome X) places people at high risk of coronary artery disease, and is characterized by a cluster of risk factors including central obesity (excessive fat tissue in the abdominal region), glucose intolerance, high triglycerides and low HDL cholesterol, and high blood pressure. Myocardial ischemia and microvascular disease is an established morbidity associated with untreated or poorly controlled metabolic syndrome.

There is a continuing need for novel antiobesity and antidiabetic agents, particularly ones that are well tolerated with few adverse effects.

GPR119 (previously referred to as GPR116) is a GPCR identified as SNORF25 in WO00/50562 which discloses both the human and rat receptors, US 6,468,756 also discloses the mouse receptor (accession numbers: AAN95194 (human), AAN95195 (rat) and ANN95196 (mouse)).

In humans, GPR119 is expressed in the pancreas, small intestine, colon and adipose tissue. The expression profile of the human GPR119 receptor indicates its potential utility as a target for the treatment of obesity and diabetes.

International patent applications WO2005/061489, WO2006/070208, WO2006/067532 disclose heterocyclic derivatives as GPR119 receptor agonists. International patent application WO2006/067531 discloses GPR119 receptor agonists. International patent applications WO2007/003960, WO2007/003961, WO2007/003962 and WO2007/003964, published after the priority date of the present application, also disclose GPR119 receptor agonists. Rolland et al J. Med. Chem. 2005, 48,6563-6574 relates to G-protein-coupled receptor agonists. International patent application WO03/077907 relates to CCR-3 antagonists.

The present invention relates to agonists of GPR119 which are useful for the treatment of diabetes and as peripheral regulators of satiety, e.g. for the treatment of obesity and metabolic syndrome.

### SUMMARY OF THE INVENTION

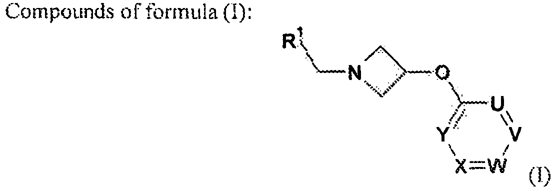

or pharmaceutically acceptable salts thereof, are agonists of GPR119 and are useful for the treatment of diabetes and as peripheral regulators of satiety, e.g. for the treatment of obesity and metabolic syndrome.

### DETAILED DESCRIPTION OF THE INVENTION

The present invention is directed to a compound of formula (I), or a pharmaceutically acceptable salt thereof: 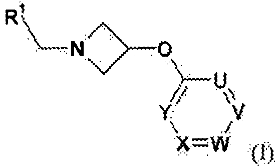 wherein: W is CR² or nitrogen;
V and X are each independently CR³ or nitrogen; U and Y are each independently CR⁴ or nitrogen, with the proviso that not more than three of U, V, W, X and Y are nitrogen;
R¹ is phenol, naphthyl, 6- to 10-membered heteroaryl, 6-membered heterocyclyl, C₃₋₈cycloalkyl, 2,3-dihydrobenzofuryl, or C₃₋₈ alkyl; any of which may be optionally substituted by up to 3 groups selected from halo, C₁₋₄ alkyl, C₂₋₄alkenyl, C₂₋₄ alkynyl, C₁₋₄ haloalkyl, (CH₂)ₘCN, C(O)NR⁹R¹⁰, C(O)R⁶, S(O)ₙR⁶, SO₂NR⁹R¹⁰, , (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy, halo, cyano, C₁₋₄ alkyl, C₁₋₄ haloalkyl, (CH₂)ₘCN, C(O)NR⁹R¹⁰, C(O)R⁶, S(O)ₙR⁶, SO₂NR⁹R¹⁰, NR¹¹C(O)NR⁹R¹⁰ or (CH₂)ₘOR⁵ groups;
R², R³ and R⁴ are independently selected from hydrogen, halo, C₁₋₄ alkyl, C₂₋₄ alkenyl, C₂₋₄ alkynyl, C₁₋₄ haloalkyl, (CH₂)ₘOR⁵, (CH₂)ₘCN, S(O)ₙR⁶, C(O)NR⁹R¹⁰, S(O)₂NR⁹R¹⁰, NR¹¹C(O)NR⁹R¹⁰, C(O)R⁶, phenyl or 5- or 6-membered heteroaryl, any of which phenyl or heteroaryl groups may be optionally substituted by halo, C₁₋₄ alkyl, C₁₋₄ alkoxy, hydroxy, C₁₋₄ haloalkyl, (CH₂)ₘCN, S(O)ₙR⁶, C(O)NR⁹R¹⁰, C(O)R⁶, NR¹¹C(O)NR⁹R¹⁰ or SO₂NR⁹R¹⁰;
or R² and an R³ group, or R³ and an adjacent R⁴ group may form a fused 6-membered aryl or nitrogen containing heteroaryl ring, either of which may be optionally substituted by halo, C₁₋₄ alkyl, C₁₋₄ haloalkyl, (CH₂)ₘCN, (CH₂)ₘOR⁵, S(O)ₙR⁶, C(O)R⁶, C(O)NR⁹R¹⁰ or SO₂NR⁹R¹⁰_{;}
R⁵ is hydrogen, C₁₋₄ alkyl, C₁₋₄ haloalkyl, (CH₂)ₘNR⁷R⁸, or (CH₂)ₘphenyl which phenyl group may be optionally substituted by halo, C₁₋₄ alkyl, C₁₋₄ alkoxy, C₁₋₄ haloalkyl or CN;
R⁶ is C₁₋₄ alkyl, optionally substituted by hydroxy or NR⁷R⁸;
R⁷ and R⁸ are independently selected from hydrogen and C₁₋₄ alkyl, or R⁷ and R⁸ may form a 5- to 7-membered heterocyclic ring optionally substituted by hydroxy or methyl;
R⁹ and R¹⁰ are independently selected from hydrogen and C₁₋₄ alkyl, optionally substituted by hydroxy or NR⁷R⁸, or, taken together, R⁹ and R¹⁰ may form a 5- or 6-membered heterocyclic ring optionally substituted by hydroxy or C₁₋₄ alkyl;
R¹¹ is hydrogen or methyl;
m is 0, 1, 2 or 3; and
n is 0, 1 or 2;
provided that when W is CR², V and X are CR³ and U and Y are CR⁴, then R¹ is not piperidin-4-yl substituted on nitrogen by phenyl or a 6-membered nitrogen containing heteroaryl.

A particular group of compounds which may be mentioned are those of formula (II), and pharmaceutically acceptable salts thereof: 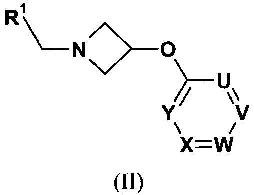 wherein:
W is CR² or nitrogen;
V and X are each independently CR³ or nitrogen; U and Y are each independently CR⁴ or nitrogen, with the proviso that not more than three of U, V, W, X and Y are nitrogen;
R¹ is phenyl, naphthyl, 6- to 10-membered heteroaryl, 6-membered heterocyclyl, C₃₋₈ cycloalkyl, 2,3-dihydrobenzofuryl, or C₃₋₈ alkyl; any of which may be optionally substituted by up to 3 groups selected from halo, C₁₋₄ alkyl, C₂₋₄ alkenyl, C₂₋₄ alkynyl, C₁₋₄ haloalkyl, (CH₂)ₘCN, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy groups;
R², R³ and R⁴ are independently selected from hydrogen, halo, C₁₋₄ alkyl, C₂₋₄ alkenyl, C₂₋₄ alkynyl, C₁₋₄ haloalkyl, (CH₂)ₘOR⁵, (CH₂)ₘCN, S(O)ₙR⁶, C(O)R⁶, phenyl or 5- or 6-membered heteroaryl, any of which phenyl or heteroaryl groups may be optionally substituted by halo, C₁₋₄alkyl, C₁₋₄ haloalkyl, (CH₂)ₘCN, S(O)ₙR⁶, C(O)NR⁹R¹⁰ or SO₂NR⁹R¹⁰;
or R² and an R³ group, or R³ and an adjacent R⁴ group may form a fused 6-membered aryl or nitrogen containing heteroaryl ring, either of which may be optionally substituted by halo, C₁₋₄ alkyl, C₁₋₄ haloalkyl, (CH₂)ₘCN, (CH₂)ₘOR⁵, S(O)ₙR⁶, C(O)NR⁹R¹⁰ or SO₂NR⁹R¹⁰;
R⁵ is hydrogen, C₁₋₄ alkyl, C₁₋₄ haloalkyl, (CH₂)ₘphenyl or (CH₂)ₘNR⁷R⁸;
R⁶ is C₁₋₄ alkyl, optionally substituted by hydroxy;
R⁷ and R⁸ are independently selected from hydrogen and C₁₋₄ alkyl;
R⁹ and R¹⁰ are independently selected from hydrogen and C₁₋₄ alkyl, optionally substituted by hydroxy, or, taken together, R⁹ and R¹⁰ may form a 5- or 6-membered heterocyclic ring optionally substituted by hydroxy;
m is 0, 1, 2 or 3; and
n is 0, 1 or 2;
provided that when W is CR², V and X are CR³ and U and Y are CR⁴, then R¹ is not piperidin-4-yl substituted on nitrogen by phenyl or a 6-membered nitrogen containing heteroaryl.

One subgroup of compounds of particular interest are those represented by formula (III): 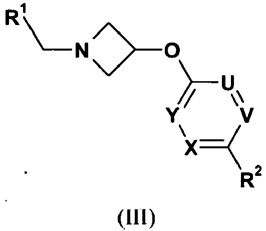 wherein:
V and X are each independently CR³ or nitrogen; U and Y are each independently CR⁴ or nitrogen, with the proviso that not more than three of U, V, X and Y are nitrogen;
R¹ is phenyl, naphthyl, 6- to 10-membered heteroaryl, 6-membered heterocyclyl, C₃₋₈ cycloalkyl, 2,3-dihydrobenzofuryl, or C₃₋₈ alkyl; any of which may be optionally substituted by up to 3 groups selected from halo, C₁₋₄ alkyl, C₂₋₄ alkenyl, C₂₋₄ alkynyl, C₁₋₄ haloalkyl, (CH₂)ₘCN, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy groups;
R² is selected from halo, C₁₋₄ alkyl, C₂₋₄ alkenyl, C₂₋₄ alkynyl, C₁₋₄ haloalkyl, (CH₂)ₘOR⁵, (CH₂)ₘCN, S(O)ₙR⁶, C(O)R⁶, phenyl or 5- or 6-membered heteroaryl, any of which phenyl or heteroaryl groups may be optionally substituted by halo, C₁₋₄ alkyl, C₁₋₄ haloalkyl, (CH₂)ₘCN, S(O)ₙR⁶, C(O)NR⁹R¹⁰ or SO₂NR⁹R¹⁰;
R³ is independently selected from hydrogen, halo, C₁₋₄ alkyl, C₂₋₄ alkenyl, C₂₋₄ alkynyl, C₁₋₄ haloalkyl, (CH₂)ₘCN, S(O)ₙR⁶, C(O)R⁶;
R⁴ is independently selected from hydrogen, halo, C₁₋₄ alkyl, C₂₋₄ alkenyl, C₂₋₄ alkynyl, C₁₋₄ haloalkyl, (CH₂)ₘOR⁵, (CH₂)ₘCN, S(O)ₙR⁶, C(O)R⁶, phenyl or 5- or 6-membered heteroaryl, any of which phenyl or heteroaryl groups may be optionally substituted by halo, C₁₋₄ alkyl, C₁₋₄ haloalkyl, (CH₂)ₘCN or S(O)ₙR⁶;
or R² and an R³ group may form a fused 6-membered aryl or nitrogen containing heteroaryl ring, either of which may be optionally substituted by halo, C₁₋₄ alkyl, C₁₋₄ haloalkyl, (CH₂)ₘCN, (CH₂)ₘOR⁵, S(O)ₙR⁶, C(O)NR⁹R¹⁰ or SO₁NR⁹R¹⁰;
R⁵ is hydrogen, C₁₋₄ alkyl, C₁₋₄ haloalkyl, (CH₂)ₘphenyl or (CH₂)ₘNR⁷R⁸;
R⁶ is C₁₋₄ alkyl, optionally substituted by hydroxy;
R⁷ and R⁸ are independently selected from hydrogen and C₁₋₄ alkyl;
R⁹ and R¹⁰ are independently selected from hydrogen and C₁₋₄ alkyl, optionally substituted by hydroxy, or, taken together, R⁹ and R¹⁰ may form a 5- or 6-membered heterocyclic ring optionally substituted by hydroxy;
m is 0, 1, 2 or 3; and
n is 0, 1 or 2;
or a pharmaceutically acceptable salt thereof;
provided that when W is CR², V and X are CR³ and U and Y are CR⁴, then R¹ is not piperidin-4-yl substituted on nitrogen by phenyl or a 6-membered nitrogen containing heteroaryl.

The molecular weight of the compounds of formula (I) and (II) is suitably less than 800, in particular less than 600, especially less than 500.

In one embodiment of the invention none of U, V, W, X and Y represent nitrogen. In a second embodiment of the invention one of U, V, W, X and Y represents nitrogen, for example X, or alternatively Y. In a third embodiment of the invention two of U, V, W, X and Y represent nitrogen, such as X and Y, U and Y, V and X or X and U. In a fourth embodiment if the invention three of U, V, W, X and Y represent nitrogen, such as X, Y and U, or X, Y and V.

R¹ is suitably phenyl, pyridyl or C₃₋₈ alkyl, in particular phenyl; optionally substituted by up to 3 groups as described above. When R¹ is substituted, it is suitably substituted by 1 or 2 groups. R¹ substituent groups of particular interest are C₁₋₄ alkyl, C₁₋₄ alkoxy, aryl, aryloxy, benzyloxy and trifluoromethoxy.

R² suitably represents phenyl or a 6-membered heteroaryl group, either of which may be optionally substituted as described above. Suitably, when substituted, R² groups are substituted by 1 or 2 groups. R² substituent groups of particular interest are (CH₂)ₘCN, for example CN and S(O)ₙR⁶,

R³ suitably represents hydrogen, C₁₋₄ alkyl or halo, especially hydrogen.

R⁴ suitably represents hydrogen, C₁₋₄ alkyl or halo, especially hydrogen.

R⁵ suitably represents C₁₋₄ alkyl, C₁₋₄ haloalkyl or (CH₂)ₘphenyl.

In one embodiment of the invention R⁶ represents C₁₋₄ alkyl. In a second embodiment of the invention R⁶ represents C₁₋₄ alkyl which is substituted by hydroxyl.

In one embodiment of the invention R⁹ and R¹⁰ independently represent hydrogen or C₁₋₄ alkyl optionally substituted by hydroxy. In a second embodiment of the invention R⁹ and R¹⁰ taken together form a 5- or 6-membered heterocyclic ring optionally substituted by hydroxy.

m is suitably 0, 1 or 2; in particular 0 or 1.

n is suitably 1 or 2.

Suitably X and V each independently represent N or CR³, e.g. N and N; CR³ and CR³; or N and CR³, wherein R³ represents hydrogen, C₁₋₄ alkyl or halo, especially hydrogen.

In one embodiment of the invention R² and an R³ group forms a fused 6-membered aryl or nitrogen containing heteroaryl ring optionally substituted by halo, C₁₋₄alkyl, C₁₋₄ haloalkyl, (CH₂)ₘCN, (CH₂)ₘOR⁵, S(O)ₙR⁶, C(O)NR⁹R¹⁰ or SO₂NR⁹R¹⁰ (e.g. R² and an R³ group forms a fused 6-membered aryl or nitrogen containing heteroaryl ring optionally substituted by halo, C₁₋₄ alkyl, C₁₋₄ haloalkyl, (CH₂)ₘCN, (CH₂)ₘOR⁵ or S(O)ₙR⁶). In an alternative embodiment of the invention R¹ and an R³ group do not form a fused 6-membered aryl or nitrogen containing heteroaryl ring.

While the preferred groups for each variable have generally been listed above separately for each variable, preferred compounds of this invention include those in which several or each variable in formula (I) is selected from the preferred, more preferred or particularly listed groups for each variable. Therefore, this invention is intended to include all combinations of preferred, more preferred and particularly listed groups.

Specific compounds of the invention which may be mentioned are those included in the Examples and pharmaceutically acceptable salts thereof.

As used herein, unless stated otherwise, "alkyl" as well as other groups having the prefix "alk" such as, for example, alkenyl, alkynyl, and the like, means carbon chains which may be linear or branched or combinations thereof. Examples of alkyl groups include methyl, ethyl, propyl (n-propyl and isopropyl), butyl (n-butyl, sec-butyl and tert-butyl), pentyl, e.g. n-pentyl, 2-ethylpropyl, 1,1-dimethylpropyl, hexyl, heptyl, octyl and the like. "Alkenyl", "alkynyl" and other like terms include carbon chains having at least one unsaturated carbon-carbon bond.

The term "haloalkyl" includes alkyl groups substituted by one or more halo atoms in particular fluorine atoms, e.g. CH₂F, CHF₂ and CF₃.

The term "cycloalkyl" means carbocycles containing no heteroatoms, and includes monocyclic saturated and partially saturated carbocycles. Examples of cycloalkyl include cyclopropyl, cyclobutyl, cyclopentyl, cyclohexyl and cycloheptyl. Examples of partially saturated cycloalkyl groups include cyclohexene. Cycloalkyl groups will typically contain 3 to 8, e.g. 3 to 6, ring carbon atoms in total.

The term "halo" includes fluorine, chlorine, bromine, and iodine atoms.

The term "aryl" includes phenyl and naphthyl, in particular phenyl.

Unless otherwise indicated the term "heterocyclyl" includes 5- and 6-membered monocyclic saturated and partially saturated rings containing up to three heteroatoms selected from N, O and S. Examples of heterocyclic rings include tetrahydrofuran, tetrahydropyran, tetrahydrothiophene, tetrahydrothiopyran, pyrrolidine, piperidine, [1,3]dioxane, oxazolidine, piperazine, morpholine and the like. Other examples of heterocyclic rings include the oxidised forms of the sulfur-containing rings. Thus, tetrahydrothiophene 1-oxide, tetrahydrothiophene 1,1-dioxide, tetrahydrothiopyran 1-oxide, and tetrahydrothiopyran 1,1-dioxide are also considered to be heterocyclic rings.

Unless otherwise stated, the term "heteroaryl" includes mono- and bicyclic 5- to 10-membered, e.g. monocyclic 5- or 6-membered, heteroaryl rings containing up to 4 heteroatoms selected from N, O and S. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridinyl, pyridazinyl, pyrimidinyl, pyrazinyl and triazinyl. Bicyclic heteroaryl groups include bicyclic heteroaromatic groups where a 5- or 6-membered heteroaryl ring is fused to a phenyl or another heteroaromatic group. Examples of such bicyclic heteroaromatic rings are benzofuran, benzothiophene, indole, benzoxazole, benzothiazole, indazole, benzimidazole, benzotriazole, quinoline, isoquinoline, quinazoline, quinoxaline and purine.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above formula (I) is shown without a definitive stereochemistry at certain positions. The present invention includes all stereoisomers of formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of formula (1) exists, the present invention includes any possible tautomers and pharmaceutically acceptable salts thereof, and mixtures thereof, except where specifically drawn or stated otherwise.

The compound of formula (I) and pharmaceutically acceptable salts thereof may exist in the form of solvates or polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline, *N',N'-*dibenzylethylenediamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like

Since the compounds of formula (I) are intended for pharmaceutical use they are preferably provided in substantially pure form, for example at least 60% pure, more suitably at least 75% pure (e.g. 90% or 95%), especially at least 98% pure (% are on a weight for weight basis).

The compounds of formula (I) can be prepared as described below, in which the variable groups are as defined above.

Certain compounds of formula (I) can be made as outlined in Scheme 1. Azetidine 1 is commercially available or can be prepared as outlined in Syn. Comm., 33(24), 4297-4302; 2003. Azetidine 2 can be prepared by treatment of 1 with a hydrogen source such as triethylamine and formic acid, in a solvent such as ethanol in the presence of palladium on carbon. Compounds of type 4 can be prepared by reductive amination of an aldehyde 3 using a suitable reducing agent such as sodium triacetoxyborohydride. The hydroxy group can be converted into a leaving group such as methanesulfonyl, allowing, in the presence of a base, displacement with a phenol 6 to afford the compound of formula (I). Alternatively, a compound of type 4 could be converted into an azetidine of formula (1) directly, via a Mitsonobu reaction with the corresponding phenol 6 by standard techniques.

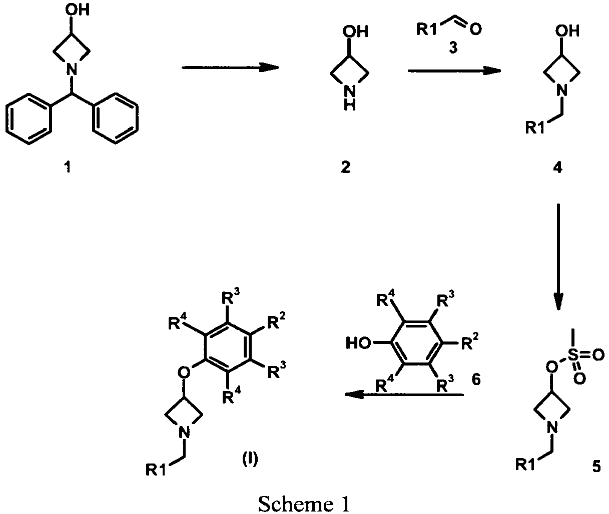

Compounds of type formula (1) can also be prepared as outlined in Scheme 2. Thus, an azetidine of type 8, incorporating a nitrogen protecting group, in this case 2,4-dimethoxybenzyl, can be converted to a compound of formula 10 via an activated azetidine of type 9. Removal of the nitrogen protecting group using standard techniques affords a compound of formula 11. Reductive amination of 11 with an aldehyde of type 3 using a suitable reducing agent affords the compound of formula (1).

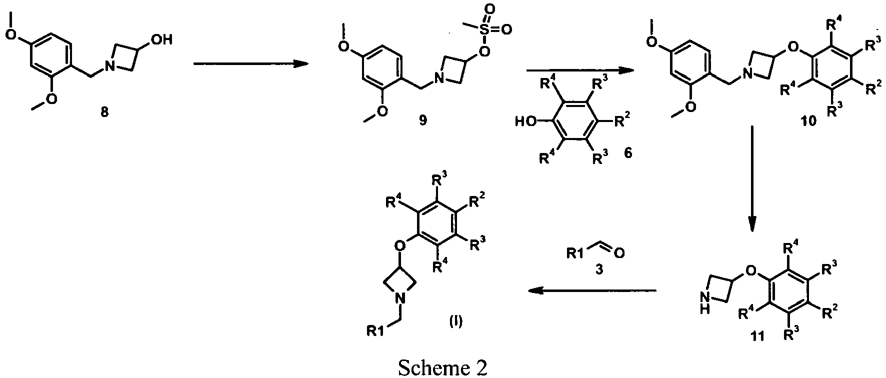

Other compounds of formula (I) can be prepared as outlined in Scheme 3. Thus reaction of compound 4 with a 2-halopyridine such as 12 in the presence of a base affords compounds of formula (I). In a further example, where X is a halogen, using readily known coupling methods 14 can be reacted with a boronic ester, or boronic acid of formula 15 to afford a compound of type 16.

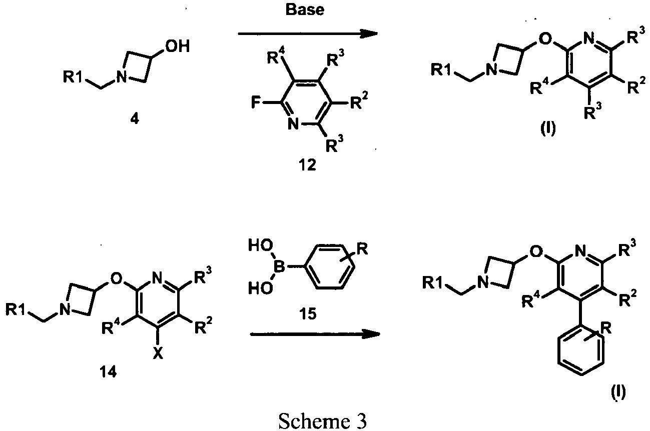

Phenols of type 19 can be prepared using standard Suzuki coupling methods as outlined in Scheme 4. Thus an aryl boronic ester or acid of type 17 can be coupled with an arylhalide of type 18 in the presence of a suitable base and palladium catalyst in an appropriate solvent or solvent mixture. Alternatively, the arylhalide could incorporate the hydroxy substituent. The aryl boronic acid or aryl halide could also be substituted for a heteroaromatic.

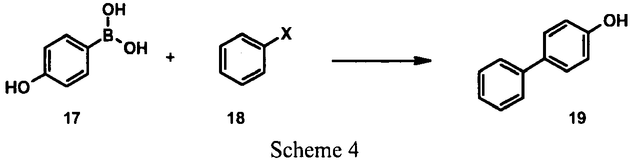

Fluoropyridines of type 22 can similarly be prepared using standard Suzuki coupling methods, as outlined in Scheme 5. Thus, an aryl boronic ester or acid of type 20 could be coupled with a dihalopyridine such as 21 to obtain a fluoropyridine of type 22.

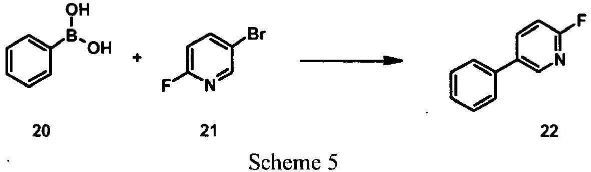

Other compounds of formula (I) may be prepared by methods analogous to those described above or by methods known *per se.*

Further details for the preparation of the compounds of formula (I) are found in the examples.

The compounds of formula (I) may be prepared singly or as compound libraries comprising at least 2, for example 5 to 1,000, compounds and more preferably 10 to 100 compounds of formula (I). Compound libraries may be prepared by a combinatorial "split and mix" approach or by multiple parallel synthesis using either solution or solid phase chemistry, using procedures known to those skilled in the art.

During the synthesis of the compounds of formula (I), labile functional groups in the intermediate compounds, e.g. hydroxy, carboxy and amino groups, may be protected. The protecting groups may be removed at any stage in the synthesis of the compounds of formula (I) or may be present on the final compound of formula (I). A comprehensive discussion of the ways in which various labile functional groups may be protected and methods for cleaving the resulting protected derivatives is given in, for example, Protective Groups in Organic Chemistry, T.W. Greene and P.G.M. Wuts, (1991) Wiley-Interscience, New York, 2nd edition.

Any novel intermediates, such as those defined above, may be of use in the synthesis of compounds of formula (I).

As indicated above the compounds of formula (I) are useful as GPR119 agonists, e.g. for the treatment and/or prophylaxis of obesity and diabetes. For such use the compounds of formula (1) will generally be administered in the form of a pharmaceutical composition.

The invention also provides a compound of formula (I), or a pharmaceutically acceptable salt thereof, for use as a pharmaceutical.

The invention also provides a pharmaceutical composition comprising a compound of formula (I), in combination with a pharmaceutically acceptable carrier.

Preferably the composition is comprised of a pharmaceutically acceptable carrier and a non-toxic therapeutically effective amount of a compound of formula (I), or a pharmaceutically acceptable salt thereof.

Moreover, the invention also provides a pharmaceutical composition for the treatment of disease by modulating GPR119, resulting in the prophylactic or therapeutic treatment of obesity, e.g. by regulating satiety, or for the treatment of diabetes, comprising a pharmaceutically acceptable carrier and a non-toxic therapeutically effective amount of compound of formula (I), or a pharmaceutically acceptable salt thereof.

The pharmaceutical compositions may optionally comprise other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds of formula (I), or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous).

Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound of formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

The compounds of formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient.

For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, using a compound of formula (I), or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of formula (I), or pharmaceutically acceptable salts thereof, may also be prepared in powder or liquid concentrate form.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of formula (I), or pharmaceutically acceptable salts thereof, may also be prepared in powder or liquid concentrate form.

Generally, dosage levels on the order of 0.01mg/kg to about 150mg/kg of body weight per day are useful in the treatment of the above-indicated conditions, or alternatively about 0.5mg to about 7g per patient per day. For example, obesity may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of formula (I) may be used in the treatment of diseases or conditions in which GPR119 plays a role. Thus the invention also provides a compound of formula (I), or a pharmaceutically acceptable salt thereof for use in the treatment of a disease or condition in which GPR119 plays a role.

Diseases or conditions in which GPR119 plays a role include obesity and diabetes. In the context of the present application the treatment of obesity is intended to encompass the treatment of diseases or conditions such as obesity and other eating disorders associated with excessive food intake e.g. by reduction of appetite and body weight, maintenance of weight reduction and prevention of rebound and diabetes (including Type 1 and Type 2 diabetes, impaired glucose tolerance, insulin resistance and diabetic complications such as neuropathy, nephropathy, retinopathy, cataracts, cardiovascular complications and dyslipidaemia). And the treatment of patients who have an abnormal sensitivity to ingested fats leading to functional dyspepsia. The compounds of the invention may also be used for treating metabolic diseases such as metabolic syndrome (syndrome X), impaired glucose tolerance, hyperlipidemia, hypertriglyceridemia, hypercholesterolemia, low HDL levels and hypertension.

The invention also provides a a compound of formula (I), or a pharmaceutically acceptable salt thereof for use in the regulation of satiety.

The invention also provides a a compound of formula (I), or a pharmaceutically acceptable salt thereof for use in the treatment of obesity.

The invention also provides a compound of formula (I), or a pharmaceutically acceptable salt thereof for use in the treatment of diabetes, including type 1 and type 2 diabetes, particularly type 2 diabetes.

The invention also provides a compound of formula (I), or a pharmaceutically acceptable salt thereof for use in the treatment of metabolic syndrome (syndrome X), impaired glucose tolerance, hyperlipidemia, hypertriglyceridemia, hypercholesterolemia, low HDL levels or hypertension.

The invention also provides the use of a compound of formula (I), or a pharmaceutically acceptable salt thereof, in the manufacture of a medicament for the treatment of a condition as defined above.

In the methods of the invention the term "treatment" includes both therapeutic and prophylactic treatment.

The compounds of formula (I) may exhibit advantageous properties compared to known GPR119 agonists, for example, the compounds may exhibit improved solubility thus improving absorption properties and bioavailability, or other advantageous properties for compounds to be used as pharmaceutical.

The compounds of formula (I), or pharmaceutically acceptable salts thereof, may be administered alone or in combination with one or more other therapeutically active compounds. The other therapeutically active compounds may be for the treatment of the same disease or condition as the compounds of formula (I) or a different disease or condition. The therapeutically active compounds may be administered simultaneously, sequentially or separately.

The compounds of formula (I) may be administered with other active compounds for the treatment of obesity and/or diabetes, for example insulin and insulin analogs, gastric lipase inhibitors, pancreatic lipase inhibitors, sulfonyl ureas and analogs, biguanides, α2 agonists, glitazones, PPAR-γ agonists, mixed PPAR-α/γ agonists, RXR agonists, fatty acid oxidation inhibitors, α-glucosidase inhibitors, β-agonists, phosphodiesterase inhibitors, lipid lowering agents, glycogen phosphorylase inhibitors, antiobesity agents e.g. pancreatic lipase inhibitors, MCH-1 antagonists and CB-1 antagonists (or inverse agonists), amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, serotonergic/dopaminergic antiobesity drugs, reuptake inhibitors e.g. sibutramine, CRF antagonists, CRF binding proteins, thyromimetic compounds, aldose reductase inhibitors, glucocorticoid receptor antagonists, NHE-1 inhibitors or sorbitol dehydrogenase inhibitors.

Combination therapy comprising the administration of a compound of formula (I), or a pharmaceutically acceptable salt thereof, and at least one other antiobesity, agent represents a further aspect of the invention.

The invention also provides the use of a compound of formula (I), or a pharmaceutically acceptable salt thereof, in the manufacture of a medicament for use in combination with another antiobesity agent, for the treatment of obesity.

The compound of formula (I), or a pharmaceutically acceptable salt thereof, and the other antiobesity agent(s) may be co-administered or administered sequentially or separately.

Co-administration includes administration of a formulation which includes both the compound of formula (I), or a pharmaceutically acceptable salt thereof, and the other antiobesity agent(s), or the simultaneous or separate administration of different formulations of each agent. Where the pharmacological profiles of the compound of formula (I), or a pharmaceutically acceptable salt thereof, and the other antiobesity agent(s) allow it, coadministration of the two agents may be preferred.

The invention also provides the use of a compound of formula (1), or a pharmaceutically acceptable salt thereof, and another antiobesity agent in the manufacture of a medicament for the treatment of obesity.

The invention also provides a pharmaceutical composition comprising a compound of formula (I), or a pharmaceutically acceptable salt thereof, and another antiobesity agent, and a pharmaceutically acceptable carrier. The invention also encompasses the use of such compositions in the methods described above.

GPR119 agonists are of particular use in combination with centrally acting antiobesity agents.

The other antiobesity agent for use in the combination therapies according to this aspect of the invention is preferably a CB-1 modulator, e.g. a CB-1 antagonist or inverse agonist. Examples of CB-1 modulators include SR141716 (rimonabant) and SLV-319 ((4*S*)-(-)-3-(4-chlorophenyl)-*N*-methyl-*N*-[(4-chlorophenyl)sulfonyl]-4-phenyl-4,5-dihydro-1H-pyrazole-1-carboxamide); as well as those compounds disclosed in EP576357, EP656354, WO 03/018060, WO 03/020217, WO 03/020314, WO 03/026647, WO 03/026648, WO 03/027076, WO 03/040105, WO 03/051850, WO 03/051851, WO 03/053431, WO 03/063781, WO 03/075660, WO 03/077847, WO 03/078413, WO 03/08-190, WO 03/082191, WO 03/082833, WO 03/084930, WO 03/084943, WO 03/086288, WO 03/087037, WO 03/088968, WO 04/012671, WO 04/013120, WO 04/026301, WO 04/029204, WO 04/034968, WO 04/035566, WO 04/037823 WO 04/052864, WO 04/058145, WO 04/058255, WO 04/060870, WO 04/060888, WO 04/069837, WO 04/069837, WO 04/072076, WO 04/072077, WO 04/078261 and WO 04/108728, and the references disclosed therein.

Other diseases or conditions in which GPR119 has been suggested to play a role include those described in WO 00/50562 and US 6,468,756, for example cardiovascular disorders, hypertension, respiratory disorders, gestational abnormalities, gastrointestinal disorders, immune disorders, musculoskeletal disorders, depression, phobias, anxiety, mood disorders and Alzheimer's disease.

The invention will now be described by reference to the following examples which are for illustrative purposes and are not to be construed as a limitation of the scope of the present invention.

### EXAMPLES

### LCMS protocol:

Waters Xterra MS C18, 5µm (4.6 x 50mm, flow rate 1.5mL/min) eluting with a H₂O-MeCN gradient containing 0.1% v/v ammonia over 12min with UV detection at 215 and 254nm. Gradient information: 0.0 - 8.0min: Ramp from 95% H₂O-5% MeCN to 5% H₂O-95% MeCN; 8.0 - 9.9min: Hold at 5% H₂O-95% MeCN; 9.9 - 10.0min: Return to 95% H₂O-5% MeCN; 10.0 - 12.0min: Hold at 95% H₂O-5% MeCN. Mass spectra were obtained using an electrospray ionization source in either the positive (ESI⁺) or negative (ESI⁻) mode.

### Prep LCMS Protocol:

The gradient used for mass directed HPLC purification was as follows: Waters Xterra MS C18, 5µm (19 x 50mm, flow rate 25mL/min) eluting with a H₂O-MeCN gradient containing 0.1% v/v ammonia over 10min with UV detection at 215 and 254nm. 0.0 - 0.50min: Hold at 95% H₂O-5% MeCN; 0.5 - 7.5min: Ramp from 95% H₂O-5% MeCN to 5% H₂O-95% MeCN; 7.5 - 8.4min: Hold at 5% H₂O-95% MeCN; 8.4 - 8.5min: Return to 95% H₂O-5% MeCN; 8.5 - 10.0min: Hold at 95% H₂O-5% MeCN. Mass ions were detected using an electrospray ionization source in either the positive (ESI⁺) or negative (ESI⁻) mode. 1000:1, Post column flow splitter using LC-packings Acurate flow splitter. Make-up flow, MeCN containing 0.1 % v/v formic acid, 1mL/min.

Abbreviations and acronyms: AcOH: Acetic acid; DCM: dichloromethane; HPLC: High performance liquid chromatography; LCMS: liquid chromatography-mass spectrometry; RT: retention time; rt: room temperature; SCX: Strong cation exchange chromatography.

### Preparation 1: 1-(4-Phenoxybenzyl)azetidin-3-ol

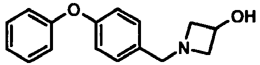

To a solution of 1-benzhydrylazetidin-3-ol (30.5g, 0.13mol) in ethanol (500mL) was added a pre-mixed solution of triethylamine (55mL, 0.39mol) and formic acid (15mL, 0.39mol) in ethanol (100mL). Palladium on carbon (2.4g) was added and the mixture heated to reflux for 3h. The mixture was cooled to rt and filtered through celite. To the filtrate was added 4-phenoxybenzaldehyde (30.4g, 0.15mol) and the mixture stirred for 10min before sodium triacetoxyborohydride (108g, 0.51mol) was added. After stirring for 24h at rt the mixture was poured into 1M HCl and washed with diethyl ether. The aqueous mixture was neutralized with a saturated solution of NaHCO₃ and the product extracted with DCM. The DCM solution was dried (MgSO₄) and evaporated to afford the title compound: RT = 5.09min; m/z (ES⁺) = 256.16 [M+H]⁺.

### Preparation 2: 1-(4-Phenoxybenzyl)azetidin-3-yl methanesulfonate

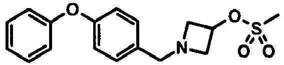

To a solution of 1-(4-phenoxybenzyl)azetidin-3-ol (2g, 7.8mmol) and triethylamine (Preparation 1, 2.4mL, 17.0mmol) in DCM (30mL) at -40°C, under N₂, was added methanesulfonyl chloride (0.75mL, 9.7mmol). After stirring for 30min at -40°C the mixture was allowed to warm to rt. The mixture was diluted with diethyl ether (400mL) and washed with saturated NaHCO₃ solution followed by brine. The organic solution was dried (MgSO₄) and evaporated, to afford the title compound: RT = 5.85min; m/z (ES⁺) = 334.11 [M+H]⁺.

### Preparation 3: 4-(6-(Methylsulfonyl)pyridin-3-yl)phenol

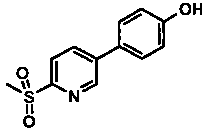

A microwave tube was charged with 5-bromo-2-methylsulfonylpyridine (3g, 12.7mmol), 4-phenol boronic acid (2.1g, 15.3mmol), Na₂CO₃ (3M solution in water, 5.1mL, 15.3mmol), [1,1'-bis(diphenylphosphino)ferrocene]dichloropalladium(II) (0.9g, 1.3mmol) and 4:1, dimethoxyethane:ethanol (30mL). The mixture was heated at 120°C in a microwave reactor for 20min. The reaction mixture was allowed to cool to rt before it was filtered through celite, washing through with methanol. The filtrate was evaporated and the crude material purified by flash chromatography eluting with 40% - 100% ethyl acetate in hexanes. The product was further purified by triturating with ethyl acetate to afford the title compound: RT = 1.82min; m/z (ES⁺) = 250.07 [M+H]⁺.

### Preparation 4: 5-Bromo-2-(1-(4-phenoxybenzyl)azetidin-3-yloxy)pyridine

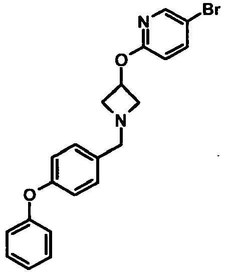

A mixture of 1-(4-phenoxybenzyl)azetidin-3-ol (Preparation 1, 200mg), 5-bromo-2-fluoropyridine (166mg) and potassium *tert*-butoxide (106mg) in tetrahydrofuran (2mL) was shaken at rt for 15h. The reaction mixture was diluted with ethyl acetate and washed with water. The organic solution was dried (MgSO₄) and evaporated to afford the title compound: RT = 6.55min; m/z (ES⁺) = 410.92 [M+H]⁺.

### Preparation 5: 1-(2,4-Dimethoxybenzyl)azetidin-3-ol

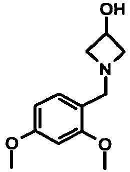

A solution of triethylamine (35mL, 251mmol) and formic acid (9.5mL, 251mmol) in ethanol (100mL) was added to a solution of 1-benzhydrylazetidin-3-ol (20g, 84mmol) in ethanol (250mL). Palladium on carbon (1g) was added and the mixture heated to reflux for 1h. The mixture was allowed to cool slightly before being filtered through celite and washing through with ethanol. The filtrate was transferred to a 2L round bottomed flask and 2,4-dimethoxybenzaldehyde (16.7g, 100mmol) added. After stirring at rt for 10min sodium triacetoxyborohydride (71g, 335mmol) was added and the mixture was stirred at rt overnight. The reaction was quenched by the addition of 1M HCl and then washed with diethyl ether. The aqueous phase was neutralized with NaHCO₃ and extracted with DCM. LCMS analysis showed that the desired product was mainly contained in the aqueous phase and could not be extracted with DCM or ethyl acetate. The aqueous phase was evaporated and the residue was stirred in a mixture of DCM (250mL), ethyl acetate (250mL) and methanol (100mL). The suspension was filtered and the filtrate evaporated to yield the crude product. The crude product was stirred in ethyl acetate, the insoluble material filtered off, and the filtrate evaporated to afford the title compound: RT = 3.22min; m/z (ES⁺) = 224.16 [M+H]⁺.

### Preparation 6: 1-(2,4-Dimethoxybenzyl)azetidin-3-yl methanesulfonate

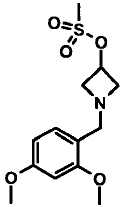

A solution of 1-(2,4-dimethoxybenzyl)azetidin-3-ol (Preparation 5, 10g, 44.8mmol) and triethylamine (14mL, 98.7mmol) in DCM (200mL) was cooled to -40°C before methanesulfonyl chloride (4.2mL, 53.8mmol) was added dropwise. The mixture was stirred at -40°C for 20min then diluted with DCM and washed with saturated NaHCO₃ solution followed by brine. The organic solution was dried (MgSO₄) and concentrated to afford the title compound: RT = 3.94 min; m/z (ES⁺) = 302.03 [M+H]⁺.

### Preparation 7: 5-(4-(1-(2,4-Dimethoxybenzyl)azetidin-3-yloxy)phenyl)-2-(methylsulfonyl)pyridine

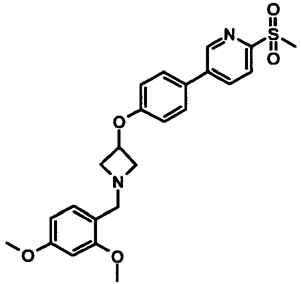

Potassium-*tert*-butoxide (7.55g, 67.3mmol) was added to a solution of 4-(6-(methylsulfonyl)pyridin-3-yl)phenol (Preparation 3, 13.4g, 53.8mmol) in dimethyl sulfoxide (50mL). The mixture was stirred at rt for 20min before a solution of 1-(2,4-dimethoxybenzyl)azetidin-3-yl methanesulfonate (14.2g, 95%, 44.8mmol) in dimethyl sulfoxide (25mL) was added. The mixture was heated at 60°C for 1h. The mixture was allowed to cool to rt then partitioned between DCM and brine. The organic phase was further washed with brine. The combined brine washings were extracted with diethyl ether. The combined organics were dried (MgSO₄) and evaporated. Purification via SCX afforded the title compound: RT = 5.00min; m/z (ES⁺) = 454.98 [M+H]⁺.

### Preparation 8: 2,2,2-Trifluoro-1-(3-(4-(6-(methylsulfonyl)pyridin-3-yl)phenoxy)azetidin-1-yl)ethanone

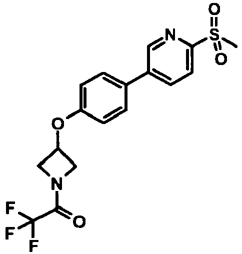

Trifluoroacetic anhydride (4.2mL, 29.6mmol) was slowly added to a cooled (ice-bath) solution of 5-(4-(1-(2,4-dimethoxybenzyl)azetidin-3-yloxy)phenyl)-2-(methylsulfonyl)pyridine (Preparation 7, 6.7g, 14.8mmol) and triethylamine (4.6mL, 32.6mmol) in DCM (70mL). After stirring with cooling for 30min the reaction mixture was diluted with DCM and washed with saturated NaHCO₃ solution. The organic solution was dried (MgSO₄), evaporated and purified via flash chromatography to afford the title compound: RT = 4.45min; m/z (ES⁺) = 400.84 [M+H]⁺.

### Preparation 9: 5-(4-(Azetidin-3-yloxy)phenyl)-2-(methylsulfonyl)pyridine

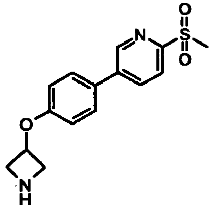

Potassium carbonate (1.5g, 11.1mmol) was added to a suspension of 2,2,2-trifluoro-1-(3-(4-(6-(methylsulfonyl)pyridin-3-yl)phenoxy)azetidin-1-yl)ethanone (Preparation 8, 3.0g, 7.4mmol) in a mixture of methanol (60mL) and water (6mL). The mixture was stirred at rt for 1h. The mixture was evaporated and the residue was stirred with 20% methanol in DCM. The mixture was filtered and the filtrate flushed through a silica plug eluting with further 20% methanol in DCM, then 100% methanol to afford the title compound: RT = 3.62min; m/z (ES⁺) = 305.02 [M+H]⁺.

### Preparation 10: 4-(4-Hydroxyphenyl)picolinonitrile

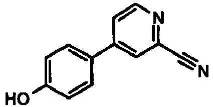

A mixture of 4-bromo-2-cyanopyridine (2.05g, 11.21mmol), 4-(4,4,5,5-tetramethyl-1,3,2-dioxaborolan-2-yl)phenol (2.47g, 11.21mmol), 3M Na₂CO₃ (3.74mL, 11.21mmol) and [1,1'-bis(diphenylphosphino)ferrocene]dichloropalladium(II) (0.82g, 1.12mmol) in 4:1, ethylene glycol dimethylether : ethanol (10mL) was reacted in the microwave at 100°C. The progress of the reaction was monitored by LCMS. The reaction mixture was filtered through celite and the filtrate evaporated. The crude material was purified by flash chromatography, followed by trituration with diethyl ether to afford the title compound: RT = 2.07min; m/z (ES⁺) = 197.07 [M+H]⁺.

The following compounds were prepared by methods analogous to those described above:
Preparation 11: 4'-Methanesulfonylbiphenyl-4-ol
Preparation 12: 4-(5-Methanesulfonylpyridin-2-yl)phenol
Preparation 13: 3-(6-Hydroxypyridin-3-yl)benzonitrile
Preparation 14: 4-(6-Methanesulfonylpyridin-3-yl)phenol
Preparation 15: 4'-Hydroxybiphenyl-3-carbonitrile
Preparation 16: 3-(5-Hydroxypyridin-2-yl)benzonitrile
Preparation 17: 4-(2-Methylpyridin-4-yl)phenol
Preparation 18: 6-(4-Methanesulfonylphenyl)pyridin-3-ol
Preparation 19: 6-m-Tolylpyridin-3-ol
Preparation 20: 2-Fluoro-5-(4-methanesulfonylphenyl)pyridine
Preparation 21: 6'-Fluoro-5-methanesulfonyl-[2,3']bipyridinyl
Preparation 22: 6-Fluoro-[3,4']bipyridin-2'-carbonitrile
Preparation 23: 6-Fluoro-6'-methanesulfonyl-[3,3']bipyridinyl.

### Examples

### General Method A

### Preparation of 4-(4-(1-(4-phenoxybenzyl)azetidin-3-yloxy)phenyl)picolinonitrile

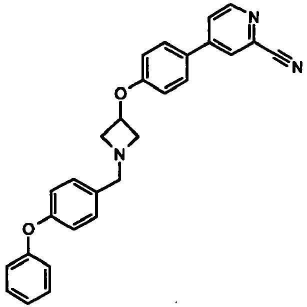

A mixture of 4-(4-hydroxyphenyl)picolinonitrile (92mg, 0.47mmol) and potassium *tert-*butoxide (53mg, 0.47mmol) in dimethyl sulfoxide (0.75mL) was shaken at rt for 15min before 1-(4-phenoxybenzyl)azetidin-3-yl methanesulfonate (Preparation 2, 78mg, 0.23mmol) was added. The reaction mixture was shaken at 60°C for 15h. The crude mixture was acidified with AcOH and purified by reverse phase HPLC purification to afford the title compound: RT = 6.75 min; m/z (ES⁺) = 434.02 [M+H]⁺.

### General Method B

### Preparation of 3-(6-(1-(4-phenoxybenzyl)azetidin-3-yloxy)pyridin-3-yl)benzonitrile

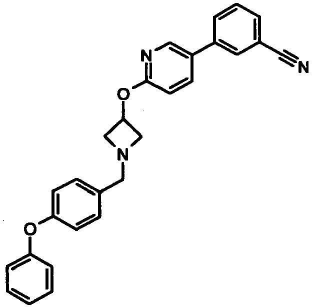

5-Bromo-2-(1-(4-phenoxybenzyl)azetidin-3-yloxy)pyridine (Preparation 4, 103mg), 3-cyanophenylboronic acid (44mg), [1,1'-bis(diphenylphosphino)ferrocene]dichloropalladium(II) (18mg) and 3M Na₂CO₃ (0.1mL) were mixed together in 4:1, ethylene glycol dimethylether : ethanol (0.6mL) and heated in a microwave reactor at 100°C for 10min. The reaction mixture was filtered through celite, acidified with AcOH and purified by reverse phase HPLC purification to afford the title compound: RT = 6.97min; m/z (ES⁺) = 434.04 [M+H]⁺.

### General Procedure C

### Preparation of 5-(methylsulfonyl)-6'-(1-(4-phenoxybenzyl)azetidin-3-yloxy)-2,3'-bipyridine

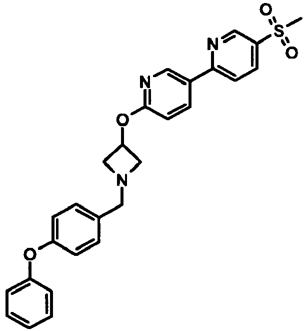

A mixture of 1-(4-phenoxybenzyl)azetidin-3-ol (Preparation 1, 60mg, 0.235mmol), 6'-fluoro-5-(methylsulfonyl)-2,3'-bipyridine (71mg, 0.282mmol) and potassium tert-butoxide (32mg, 0.282mmol) in dimethyl sulfoxide (0.5mL) was shaken at rt for 15h. The reaction mixture was acidified with AcOH and purified by reverse phase HPLC purification to afford the tile compound: RT = 6.07min; m/z (ES⁺) = 488.06 [M+H]⁺.

### General Procedure D

### Preparation of 2-methanesulfonyl-5-{4-[1-(6-methoxypyridin-3-ylmethyl)azetidin-3-yloxy]phenyl}pyridine

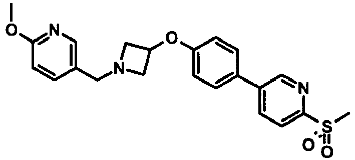

A mixture of 5-(4-(azetidin-3-yloxy)phenyl)-2-(methylsulfonyl)pyridine (Preparation 9, 97mg, 62%,0.197mmol) and 6-methoxynicotinaldehyde (32.5mg, 0.237mmol) in DCM (1mL) was shaken at rt. After 10min sodium triacetoxyborohydride (84mg, 0.395mmol) was added. After 2h the mixture was diluted with DCM (1mL) and water (0.25mL) added. The phases were separated using phase separating cartridges and the organic solution evaporated. The crude product was purified by reverse phase HPLC purification to afford the title compound: RT = 5.29min; m/z (ES⁺) = 425.98 [M+H]⁺.
The following compounds were prepared using the general methods outlined above:

| **Ex** | **Structure** | **Name** | **Method** | **RT** | **m/z** |
|---|---|---|---|---|---|
| 1 | 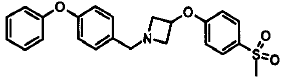 | 3-(4-Methanesulfonyl-phenoxy)-1-(4-phenoxybenzyl)azetidine | A | 6.72 | 410.00 |
| 2 | 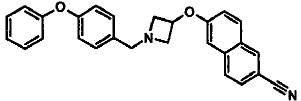 | 2 6-[1-(4-Phenoxy-benzyl)azetidin-3-yloxy]naphthalene-2-carbonitrile | A | 8.28 | 407.00 |
| 3 | 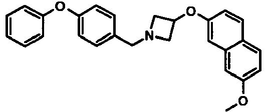 | 3-(7-Methoxynaphthalen-2-yloxy)-1-(4-phenoxy-benzyl)azetidine | A | 8.62 | 412.04 |
| 4 | 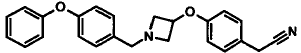 | {4-[1-(4-Phenoxy-benzyl)azctidin-3-yloxy]phenyl}acetonitrile | A | 7.28 | 371.07 |
| 5 | 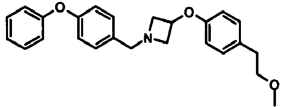 | 3-[4-(2-Methoxy-ethyl)phenoxy]-1-(4-phenoxybenzyl)azetidine | A | 7.89 | 390.08 |
| 6 | 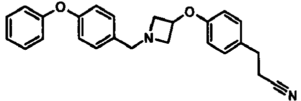 | 3-{4-[1-(4-Phenoxy-benzyl)azetidin-3-yloxy]-phenyl}propionitrile | A | 7.40 | 385.11 |
| 7 | 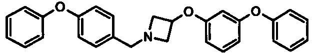 | 1-(4-Phenoxybenzyl)-3-(3-phenoxyphenoxy)-azetidine | A | 9.02 | 424.04 |
| 8 | 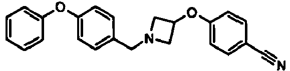 | 4-[1-(4-Phenoxybenzyl)-azetidin-3-yloxy]-benzonitrile | A | 7.47 | 357.10 |
| 9 | 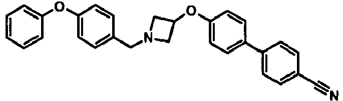 | 4'-[1-(4-Phenoxybenzyl)-azetidin-3-yloxy]-biphenyl-4-carbonitrile | A | 8.55 | 433.04 |
| 10 | 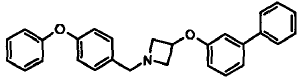 | 3-(Biphenyl-3-yloxy)-1-(4-phenoxybenzyl)-azetidine | A | 9.05 | 408.03 |
| 11 | 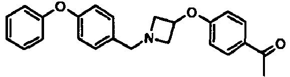 | 1-{4-[1-(4-Phenoxy-benzyl)azetidin-3-yloxy]phenyl}ethanone | A | 7.30 | 374.07 |
| 12 | 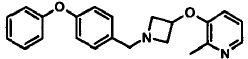 | 2-Methyl-3-[1-(4-phenoxybenzyl)azetidin-3-yloxylpyridine | A | 6.92 | 347.14 |
| 13 | 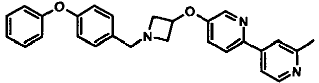 | 2'-Methyl-5-[1-(4-phenoxybenzyl)azetidin-3-yloxy]-[2,4']bipyridinyl | A | 6.20 | 424.06 |
| 14 | 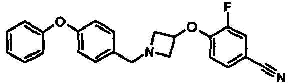 | 3-Fluoro-4-[1-(4-phenoxybenzyl)azetidin-3-yloxy]benzonitrile | A | 7.53 | 375.02 |
| 15 | 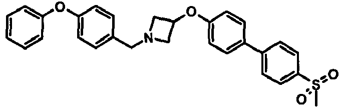 | 3-(4'-Methanesulfonyl-biphenyl-4-yloxy)-1-(4-phenoxybenzyl)azetidine | A | 6.60 | 486.02 |
| 16 | 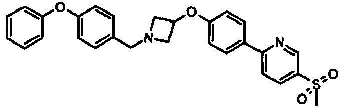 | 5-Methanesulfonyl-2-{4-[1-(4-phenoxybenzyl)-azetidin-3-yloxy]-phenyl}pyridine | A | 6.32 | 487.03 |
| 17 | 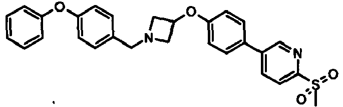 . | 2-Methanesulfonyl-5-{4-[1-(4-phenoxybenzyl)-azetidin-3-yloxy]-phenyl}pyridine | A | 6.28 | 487.04 |
| 18 | 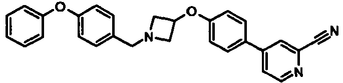 | 4-{4-[1-(4-Phenoxy-benzyl)azetidine-3-yloxy]phenyl}pyridine-2-carbonitrile | A | 6.75 | 434.02 |
| 19 | 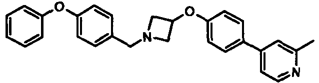 | 2-Methyl-4-{4-[1-(4-phenoxybenzyl)azetidin-3-yloxy]phenyl}pyridine | A | 6.80 | 423.04 |
| 20 | 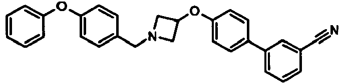 | 4'-[1-(4-Phenoxy-benzyl)azetidin-3-yloxy]-biphenyl-3-carbonitrile | A | 7.30 | 433.07 |
| 21 | 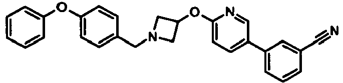 | 3-{6-[1-(4-Phenoxy-benzyl)azetidin-3-yloxy]-pyridin-3-yl}-benzonitrile | B | 6.97 | 434.04 |
| 22 | 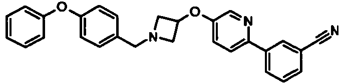 | 3-{5-[1-(4-Phenoxy-benzyl)azetidin-3-yloxy]-pyridin-2-yl}-benzonitrile | A | 6.82 | 434.02 |
| 23 | 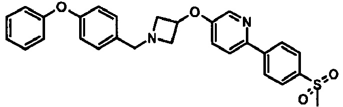 | 2-(4-Methanesulfonyl-phenyl)-5-[1-(4-phenoxybenzyl)-azetidin-3-yloxy]pyridine | A | 6.14 | 487.04 |
| 24 | 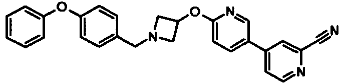 | 6-[1-(4-Phenoxybenzyl)-azetidin-3-yloxy]-[3,4']bipyridinyl-2'-carbonitrile | c | 6.45 | 435.05 |
| 25 | 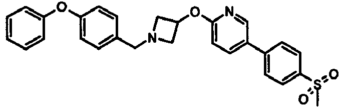 | 5-(4-Methanesulfonyl-phenyl)-2-[1-(4-phenoxybenzyl)-azetidin-3-yloxy]pyridine | c | 6.30 | 487.03 |
| 26 | 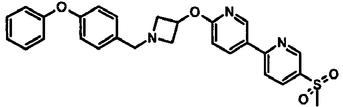 | 5-Methanesulfonyl-6'-[1-(4-phenoxybenzyl)-azetidin-3-yloxy]-[2,3']bipyridinyl | c | 6.07 | 488.06 |
| 27 | 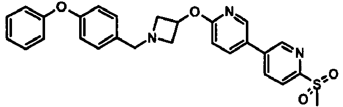 | 6'-Methanesulfonyl-6-[1-(4-phenoxybenzyl)-azetidin-3-yloxy]-[3,3']bipyridinyl | c | 5.94 | 488.06 |
| 28 | 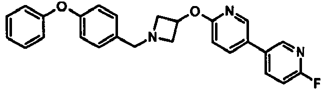 | 6'-Fluoro-6-[1-(4-phenoxybenzyl)azetidin-3-yloxy]-[3,3']bipyridinyl | B | 6.59 | 428.08 |
| 29 | 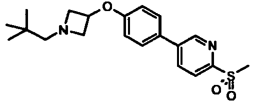 | 5-{4-[1-(2,2-Dimethyl-propyl)azetidin-3-yloxy]-phenyl}-2-methanesulfonylpyridine | D | 6.45 | 374.96 |
| 30 | 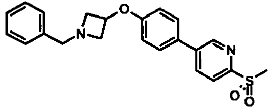 | 5-[4-(1-Benzylazetidin-3-yloxy)phenyl]-2-methanesulfonylpyridine | D | 5.93 | 394.95 |
| 31 | 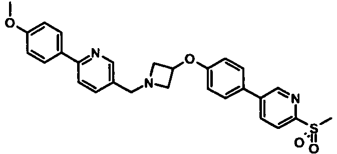 | 2-Methanesulfonyl-5-{4-[1-(6-(4-methoxyphenyl)-pyridin-3-ylmethyl)-azetidin-3-yloxy]-phenyl}pyridine | D | 5.92 | 501.99 |
| 32 | 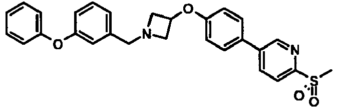 | 2-Methanesulfonyl-5-{4-[1-(3-phenoxybenzyl)-azetidin-3-yloxy]-phenyl}pyridine | D | 6.89 | 486.98 |
| 33 | 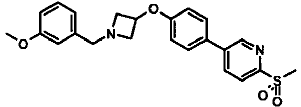 | 2-Methanesulfonyl-5-{4-[1-(3-methoxybenzyl)-azetidin-3-yloxy]-phenyl}pyridine | D | 5.90 | 424.96 |
| 34 | 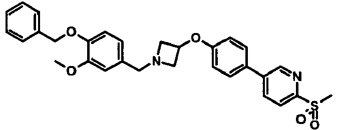 | 5-{4-[1-(4-Benzyloxy-3-methoxybenzyl)azetidin-3-yloxy]phenyl}-2-methanesulfonylpyridine | D | 6.49 | 531.06 |
| 35 | 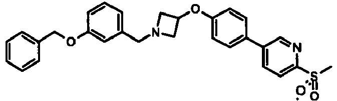 | 5-{4-[1-(3-Benzyloxy-benzyl)azetidin-3-yloxy]-phenyl}-2-methane-sulfonylpyridine | D | 6.80 | 500.98 |
| 36 | 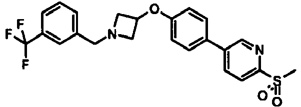 | 2-Methanesulfonyl-5-{4-[1-(3-trifluoromethyl-benzyl)azetidin-3-yloxy]phenyl}pyridine | D | 6.54 | 462.94 |
| 37 | 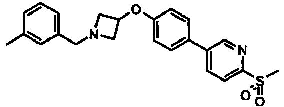 | 2-Methanesulfonyl-5-{4-[1-(3-methylbenzyl)-azetidin-3-yloxy]-phenyl}pyridine | D | 6.20 | 408.99 |
| 38 | 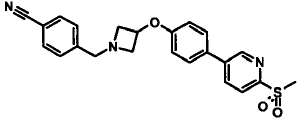 | 4-{3-[4-(6-Methanesulfonyl-pyridin-3-yl)phenoxy]azetidin-1-ylmethyl}-benzonitrile | D | 5.68 | 419.91 |
| 39 | 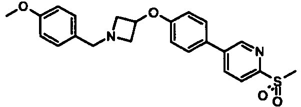 | 2-Methanesulfonyl-5-{4-[1-(4-methoxybenzyl)-azetidin-3-yloxy]-phenyl}pyridine | D | 5.80 | 424.96 |
| 40 | 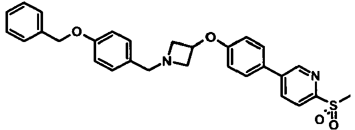 | 5-{4-[1-(4-Benzyloxy-benryl)azetidin-3-yloxy]-phenyl}-2-methane-sulfonylpyridine | D | 6.72 | 501.03 |
| 41 | 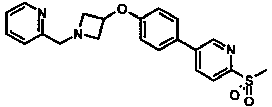 | 2-Methanesulfonyl-5-[4-(1-pyridin-2-ylmethyl-azetidin-3-yloxy)-phenyl]pyridine | D | 4.87 | 395.97 |
| 42 | 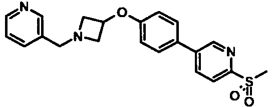 | 2-Methanesulfonyl-5-[4-(1-pyridin-3-ylmethyl-azetidin-3-yloxy)-phenyl]pyridine | D | 4.80 | 395.97 |
| 43 | 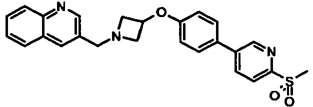 | 3-{3-[4-(6-Methane-sulfonylpyridin-3-yl)-phenoxy]azetidin-1-ymethyl}quinoline | D | 5.52 | 445.95 |
| 44 | 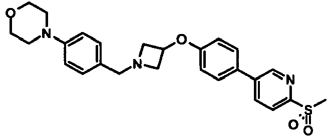 | 4-(4-{3-[4-(6-Methanesulfonylpyridin-3-yl)phenoxy]azetidin-1-ymethyl}phenyl)-morpholine | D | 5.54 | 480.01 |
| 45 | 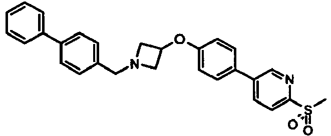 | 5-[4-(1-Biphenyl-4-yl-methylazetidin-3-yloxy)-phenyl]-2-methane-sulfonylpyridine | D | 6.79 | 471.01 |
| 46 | 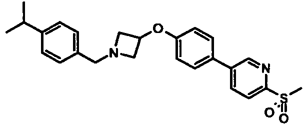 | 5-{4-[1-(4-Isopropyl-benzyl)azetidin-3-yl-oxy]phenyl}-2-methane-sulfonylpyridine | D | 6.77 | 437.03 |
| 47 | 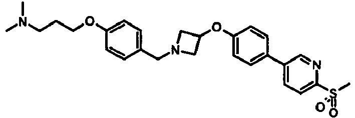 | [3-(4-{3-[4-(6-Methanesulfonylpyridin-3-yl)phenoxy]azetidin-1-ymethyl}phenoxy)-propyl]dimethylamine | D | 6.50 | 496.05 |
| 48 | 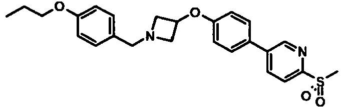 | 2-Methanesulfonyl-5-{4-[1-(4-propoxybenzyl)-azetidin-3-yloxy]-phenyl}pyridine | D | 6.54 | 452.99 |
| 49 | 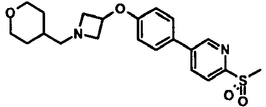 | 2-Methanesulfonyl-5-{4-[1-(tetrahydropyran-4-ylmethyl)azetidin-3-yloxy]phenyl}pyridine | D | 5.05 | 402.99 |
| 50 | 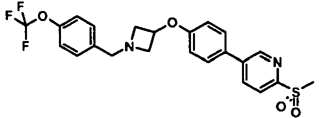 | 2-Methanesulfonyl-5-{4-[1-(4-trifluoromethoxy-benzyl)azetidin-3-yloxy]phenyl}pyridine | D | 6.60 | 478.86 |
| 51 | 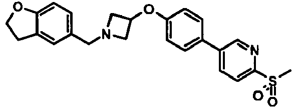 | 5-{4-[1-(2,3-Dihydro-benzofuran-5-ylmethyl)-azetidin-3-yloxy]-phenyl}-2-methane-sulfonylpyridine | D | 5.84 | 436.96 |
| 52 | 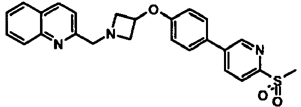 | 2-{3-[4-(6-Methane-sulfonylpyridin-3-yl)-phenoxy]azetidin-1-ymethyl}quinoline | D | 5.57 | 445.97 |
| 53 | 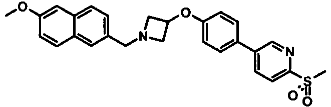 | 2-Methanesulfonyl-5-{4-[1-(6-methoxy-naphtahalen-2-ylmethyl)-azetidin-3-yloxy]-phenyl}pyridine | D | 6.45 | 474.97 |
| 54 | 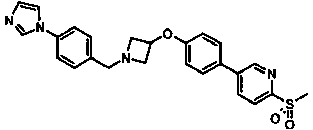 | 5-{4-[1-(4-Imidazol-1-yl-benzyl)azetidin-3-yloxy]-phenyl}-2-methane-sulfonylpyridine | D | 5.29 | 460.98 |
| 55 | 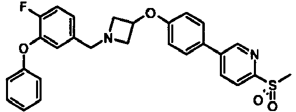 | 5-{4-[1-(4-Fluoro-3-phenoxybenzyl)azetidin-3-yloxy]phenyl}-2-methanesulfonylpyridine | D | 6.77 | 504.99 |
| 56 | 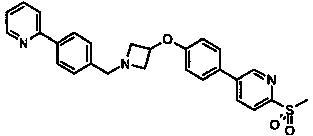 | 2-Methanesulfonyl-5-{4-[1-(4-pyridin-2-yl-benzyl)azetidin-3-yloxy]phenyl}pyridine | D | 5.87 | 471.96 |
| 57 | 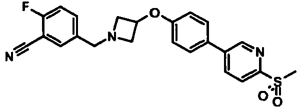 | 2-Fluoro-5-{3-[4-(6-methanesulfonylpyridin-3-yl)phenoxy]azetidin-1-ylmethyl}benzonitrile | D | 5.80 | 437.93 |
| 58 | 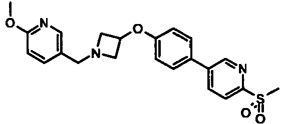 | 2-Methanesulfonyl-5-{4-[1-(6-methoxypyridin-3-ylmethyl)azetidin-3-yloxy]phenyl}pyridine | D | 5.29 | 425.98 |
| 59 | 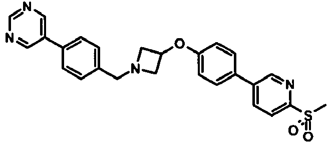 | 5-(4-{3-[4-(6-Methane-sulfonylpyridin-3-yl)-phenoxy]azetidin-1-ymethyl}phenyl)-pyrimidine | D | 5.29 | 472.99 |
| 60 | 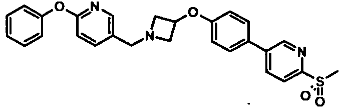 | 2-Methanesulfonyl-5-{4-[1-(6-phenoxypyridin-3-ylmethyl)azetidin-3-yloxy]phenyl}pyridine | D | 6.00 | 488.00 |
| 61 | 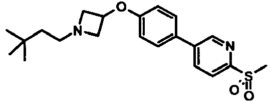 | 5-{4-[1-(3,3-Dimethyl-butyl)azetidin-3-yloxy]-phenyl}-2-methane-sulfonylpyridine | D | 6.42 | 389.02 |
| 62 | 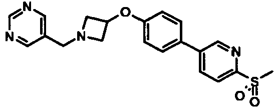 | 5-{3-[4-(6-Methane-sulfonylpyridin-3-yl)-phenoxy]azetidin-1-ymethyl}pyrimidine | D | 4.42 | 396.94 |

The biological activity of the compounds of the invention may be tested in the following assay systems:

### Yeast Reporter Assay

The yeast cell-based reporter assays have previously been described in the literature (e.g. see Miret J. J. et al, 2002, J. Biol. Chem., 277:6881-6887; Campbell R.M. et al, 1999, Bioorg. Med. Chem. Lett., 9:2413-2418; King K. et al, 1990, Science, 250:121-123; WO 99/14344; WO 00/12704; and US 6,100,042). Briefly, yeast cells have been engineered such that the endogenous yeast G-alpha (GPA1) has been deleted and replaced with G-protein chimeras constructed using multiple techniques. Additionally, the endogenous yeast alpha-cell GPCR, Ste3 has been deleted to allow for a homologous expression of a mammalian GPCR of choice. In the yeast, elements of the pheromone signaling transduction pathway, which are conserved in eukaryotic cells (for example, the mitogen-activated protein kinase pathway), drive the expression of Fus1. By placing β-galactosidase (LacZ) under the control of the Fus1 promoter (Fus1p), a system has been developed whereby receptor activation leads to an enzymatic read-out.

Yeast cells were transformed by an adaptation of the lithium acetate method described by Agatep et al, (Agatep, R. et al, 1998, Transformation of Saccharomyces cerevisiae by the lithium acetate/single-stranded carrier DNA/polyethylene glycol (LiAc/ss-DNA/PEG) protocol. Technical Tips Online, Trends Journals, Elsevier). Briefly, yeast cells were grown overnight on yeast tryptone plates (YT). Carrier single-stranded DNA (10 µg), 2 µg of each of two Fus1p-LacZ reporter plasmids (one with URA selection marker and one with TRP), 2 µg of GPR119 (human or mouse receptor) in yeast expression vector (2 µg origin of replication) and a lithium acetate/ polyethylene glycol/ TE buffer was pipetted into an Eppendorf tube. The yeast expression plasmid containing the receptor/ no receptor control has a LEU marker. Yeast cells were inoculated into this mixture and the reaction proceeds at 30°C for 60 min. The yeast cells were then heat-shocked at 42°C for 15 min. The cells were then washed and spread on selection plates. The selection plates are synthetic defined yeast media minus LEU, URA and TRP (SD-LUT). After incubating at 30°C for 2-3 days, colonies that grow on the selection plates were then tested in the LacZ assay.

In order to perform fluorimetric enzyme assays for β-galactosidase, yeast cells carrying the human or mouse GPR119 receptor were grown overnight in liquid SD-LUT medium to an unsaturated concentration (i.e. the cells were still dividing and had not yet reached stationary phase). They were diluted in fresh medium to an optimal assay concentration and 90 µL of yeast cells are added to 96-well black polystyrene plates (Costar). Compounds, dissolved in DMSO and diluted in a 10% DMSO solution to 10X concentration, were added to the plates and the plates placed at 30°C for 4 h. After 4 h, the substrate for the β-galactosidase was added to each well. In these experiments, Fluorescein di (β-D-galactopyranoside) was used (FDG), a substrate for the enzyme that releases fluorescein, allowing a fluorimetric read-out. 20 µL per well of 500 µM FDG/2.5% Triton X100 was added (the detergent was necessary to render the cells permeable). After incubation of the cells with the substrate for 60 min, 20 µL per well of 1M sodium carbonate was added to terminate the reaction and enhance the fluorescent signal. The plates were then read in a fluorimeter at 485/535nm.

The compounds of the invention gave an increase in fluorescent signal of at least ~ 1.5-fold that of the background signal (i.e. the signal obtained in the presence of 1% DMSO without compound). Compounds of the invention which give an increase of at least 5-fold may be preferred.

### cAMP Assay

A stable cell line expressing recombinant human GPR119 was established and this cell line was used to investigate the effect of compounds of the invention on intracellular levels of cyclic AMP (cAMP). The cell monolayers were washed with phosphate buffered saline and stimulated at 37°C for 30min with various concentrations of compound in stimulation buffer plus 1% DMSO. Cells were then lysed and cAMP content determined using the Perkin Elmer AlphaScreen^{™} (Amplified Luminescent Proximity Homogeneous Assay) cAMP kit. Buffers and assay conditions were as described in the manufacturer's protocol.

Compounds of the invention produced a concentration-dependent increase in intracellular cAMP level and generally had an EC₅₀ of <10 µM. Compounds showing an EC₅₀ of less than 1 µM in the cAMP assay may be preferred.

### In vivo feeding study

The effect of compounds of the invention on body weight and food and water intake may be examined in freely-feeding male Sprague-Dawley rats maintained on reverse-phase lighting. In such a test, compounds of the invention and and reference compounds are dosed by appropriate routes of administration (e.g. intraperitoneally or orally) and measurements made over the following 24 h. Rats are individually housed in polypropylene cages with metal grid floors at a temperature of 21±4°C and 55±20% humidity. Polypropylene trays with cage pads are placed beneath each cage to detect any food spillage. Animals are maintained on a reverse phase light-dark cycle (lights off for 8 h from 09.30-17.30 h) during which time the room was illuminated by red light. Animals have free access to a standard powdered rat diet and tap water during a two week acclimatization period. The diet is contained in glass feeding jars with aluminum lids. Each lid has a 3-4 cm hole in it to allow access to the food. Animals, feeding jars and water bottles are weighed (to the nearest 0.1 g) at the onset of the dark period. The feeding jars and water bottles are subsequently measured 1, 2, 4, 6 and 24 h after animals are dosed with a compound of the invention and any significant differences between the treatment groups at baseline compared to vehicle-treated controls.

Compounds of the invention showing a hypophagic effect at one or more time points at a dose of ≤ 100mg/kg may be preferred.

### Anti-diabetic effects of compounds of the invention in an in-vitro model of pancreatic beta cells (HIT-T15)

### Cell Culture

HIT-T15 cells (passage 60) can be obtained from ATCC, and cultured in RPM11640 medium supplemented with 10% fetal calf serum and 30nM sodium selenite. All experiments should be done with cells at less than passage 70, in accordance with the literature, which describes altered properties of this cell line at passage numbers above 81 (Zhang HJ, Walseth TF, Robertson RP. Insulin secretion and cAMP metabolism in HIT cells. Reciprocal and serial passage-dependent relationships. Diabetes. 1989 Jan;38(1):44-8).

### cAMP assay

HIT-T15 cells were plated in standard culture medium in 96-well plates at 100,000 cells/ 0.1 mL/ well and cultured for 24 h and the medium was then discarded. Cells were incubated for 15 min at room temperature with 100 µL stimulation buffer (Hanks buffered salt solution, 5 mM HEPES, 0.5 mM IBMX, 0.1% BSA, pH 7.4). This was discarded and replaced with compound dilutions over the range 0.001, 0.003, 0.01, 0.03, 0.1, 0.3, 1, 3, 10, 30 µM in stimulation buffer in the presence of 0.5% DMSO. Cells were incubated at room temperature for 30 min. Then 75 µL lysis buffer (5 mM HEPES, 0.3% Tween-20, 0.1 % BSA, pH 7.4) was added per well and the plate was shaken at 900 rpm for 20 min. Particulate matter was removed by centrifugation at 3000rpm for 5min, then the samples were transferred in duplicate to 384-well plates, and processed following the Perkin Elmer AlphaScreen cAMP assay kit instructions. Briefly 25 µL reactions were set up containing 8 µL sample, 5 µL acceptor bead mix and 12 µL detection mix, such that the concentration of the final reaction components is the same as stated in the kit instructions. Reactions were incubated at room temperature for 150 min, and the plate was read using a Packard Fusion instrument. Measurements for cAMP were compared to a standard curve of known cAMP amounts (0.01, 0.03, 0.1, 0.3, 1, 3, 10, 30, 100, 300, 1000 nM) to convert the readings to absolute cAMP amounts. Data was analysed using XLfit 3 software.

Representative compounds of the invention were found to increase cAMP at an EC₅₀ of less than 10 µM. Compounds showing an EC₅₀ of less than 1 µM in the cAMP assay may be preferred.

### Insulin secretion assay

HIT-T15 cells are plated in standard culture medium in 12-well plates at 106 cells/ 1 ml/well and cultured for 3 days and the medium then discarded. Cells are washed x 2 with supplemented Krebs-Ringer buffer (KRB) containing 119 mM NaCl, 4.74 mM KCl, 2.54 mM CaCl₂, 1.19 mM MgSO₄, 1.19 mM KH2PO4, 25 mM NaHCO₃, 10mM HEPES at pH 7.4 and 0.1% bovine serum albumin. Cells are incubated with 1ml KRB at 37°C for 30 min which is then discarded. This is followed by a second incubation with KRB for 30 min, which is collected and used to measure basal insulin secretion levels for each well. Compound dilutions (0, 0.1, 0.3, 1, 3, 10 uM) are then added to duplicate wells in 1ml KRB, supplemented with 5.6 mM glucose. After 30 min incubation at 37°C samples are removed for determination of insulin levels. Measurement of insulin may be done using the Mercodia Rat insulin ELISA kit, following the manufacturers instructions, with a standard curve of known insulin concentrations. For each well insulin levels are subtracted by the basal secretion level from the pre-incubation in the absence of glucose. Data may be analysed using XLfit 3 software.

Compounds showing an EC₅₀ of less than 1µM in the insulin secretion assay may be preferred.

### Oral Glucose Tolerance Tests

The effects of compounds of the invention on oral glucose (Glc) tolerance may also be evaluated, for example in male C57B1/6 or male *ob*/*ob* mice. Food may be withdrawn 5 h before administration of Glc and remain withdrawn throughout the study. Mice should have free access to water during the study. A cut may be made to the animals' tails, then blood (20 µL) may be removed for measurement of basal Glc levels 45 min before administration of the Glc load. Subsequently, the mice are weighed and dosed orally with test compound or vehicle (20% aqueous hydroxypropyl-β-cyclodextrin or 25% aqueous Gelucire 44/14) 30 min before the removal of an additional blood sample (20 µL) and treatment with the Glc load (2-5 g kg⁻¹ p.o.). Blood samples (20 µL) may then be taken 25, 50, 80, 120, and 180 min after Glc administration. The 20 µL blood samples for measurement of Glc levels are taken from the cut tip of the tail into disposable micro-pipettes (Dade Diagnostics Inc., Puerto Rico) and the sample should be added to 480 µL of haemolysis reagent. Duplicate 20 µL aliquots of the diluted haemolysed blood are then added to 180 µL of Trinders glucose reagent (Sigma enzymatic (Trinder) colorimetric method) in a 96-well assay plate. After mixing, the samples are left at room temperature for 30 min before being read against Glc standards (Sigma glucose/urea nitrogen combined standard set). Compounds of the invention of particular interest will typically result in a statistically significant reduction of the Glc excursion at doses ≤100 mg kg⁻¹ in this test.

## Claims

1. A compound of formula (I): 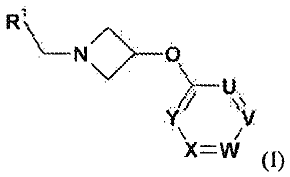 wherein:
W is CR² or nitrogen;
V and X are each independently CR³ or nitrogen; U and Y are each independently CR⁴ or nitrogen, with the proviso that not more than three of U, V, W, X and Y are nitrogen;
R¹ is phenyl, naphthyl, 6- to 10-membered heteroaryl, 6-membered heterocyclyl, C₃₋₈ cycloalkyl, 2,3-dihydrobenzofuryl, or C₃₋₈ alkyl; any of which may be optionally substituted by up to 3 groups selected from halo, C₁₋₄ alkyl, C₂₋₄ alkenyl, C₂₋₄ alkynyl, C₁₋₄ haloalkyl, (CH₂)ₘCN, C(O)NR⁹R¹⁰, C(O)R⁶, S(O)ₙR⁶, SO₂NR⁹R¹⁰, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy, halo, cyano, C₁₋₄ alkyl, C₁₋₄ haloalkyl, (CH₂)ₘCN, C(O)NR⁹R¹⁰, C(O)R⁶, S(O)ₙR⁶ SO₂NR⁹R¹⁰, NR¹¹C(O)NR⁹R¹⁰ or (CH₂)ₘOR⁵ groups;
R², R³ and R⁴ are independently selected from hydrogen, halo, C₁₋₄alkyl, C₂₋₄ alkenyl, C₂₋₄ alkynyl, C₁₋₄ Haloalkyl, (CH₂)ₘOR⁵, (CH₂)ₘCN, S(O)ₙR⁶, C(O)NR⁹R¹⁰, S(O)₂NR⁹R¹⁰, NR¹¹C(O)NR⁹R¹⁰, C(O)R⁶, phenyl or 5- or 6-membered heteroaryl, any of which phenyl or heteroaryl groups may be optionally substituted by halo, C₁₋₄ alkyl, C₁₋₄ alkoxy, hydroxy, C₁₋₄ haloalkyl, (CH₂)ₘCN, S(O)ₙR⁶, C(O)NR⁹R¹⁰, C(O)R⁶, NR¹¹C(O)NR⁹R¹⁰ or SO₂NR⁹R¹⁰;
or R² and an R³ group, or R³ and an adjacent R⁴ group may form a fused 6-membered aryl or nitrogen containing heteroaryl ring, either of which may be optionally substituted by hallo, C₁₋₄ alkyl, C₁₋₄ haloalkyl, (CH₂)ₘCN, (CH)ₘOR⁵, S(O)ₙR⁶, C(O)R⁶, C(O)NR⁹R¹⁰ or SO₂NR⁹R¹⁰.
R⁵ is hydrogen, C₁₋₄ alkyl, C₁₋₄ haloalkyl, (CH₂)ₘNR⁷R⁸, or (CH₂)ₘphenyl which phenyl group may be optionally substituted by halo, C₁₋₄ alkyl, C₁₋₄ alkoxy, C₁₋₄ haloalkyl or CN;
R⁶ is C₁₋₄ alkyl, optionally substituted by hydroxy or NR⁷R⁸;
R⁷ and R⁸ are independently selected from hydrogen and C₁₋₄ alkyl, or R⁷ and R⁸ may form a 5- to 7-membered heterocyclic ring optionally substituted by hydroxy or methyl ;
R⁹ and R¹⁰ are independently selected from hydrogen and C₁₋₄ alkyl, optionally substituted by hydroxy or NR⁷R⁸, or, taken together, R⁹ and R¹⁰ may form a 5- or 6-membered heterocyclic ring optionally substituted by hydroxy or C₁₋₄ alkyl;
R¹¹ is hydrogen or methyl;
m is 0, 1, 2 or 3; and
n is 0, 1 or 2;
or a pharmaceutically acceptable salt thereof;
provided that when W is CR², V and X are CR³ and U and Y are CR⁴, then R¹ is not piperidin-4-yl substituted on nitrogen by phenyl or a 6-membered nitrogen containing heteroaryl.

2. A compound according to claim 1, which is a compound of formula (III): 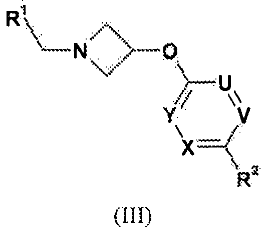 wherein:
V and X are each independently CR³ or nitrogen; U and Y are each independently CR⁴ or nitrogen, with the proviso that not more than three of U, V, X and Y are nitrogen,
R¹ is phenyl, naphthyl, 6- to 10-membered heteroaryl, 6-membered heterocyclyl, C₃₋₈ cycloalkyl, 2,3-dihydrobenzofuryl, or C₃₋₈ alkyl; any of which may be optionally substituted by up to 3 groups selected from halo, C₁₋₄ alkyl, C₂₋₄ alkenyl, C₂₋₄ alkynyl, C₁₋₄ haloalkyl, (CH₂)ₘCN, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy groups;
R² is selected from halo, C₁₋₄ alkyl, C₁₋₄ alkenyl, C₂₋₄ alkynyl, C₁₋₄ haloalkyl, (CH₂)ₘOR⁵, (CH₂)ₘCN, S(O)ₙR⁶, C(O)R⁶, phenyl or 5- or 6-membered heteroaryl, any of which phenyl, or heteroaryl groups may be optionally substituted by halo, C₁₋₄ alkyl, C₁₋₄ haloalkyl, (CH₂)ₘCN, S(O)ₙR⁶, C(O)NR⁹R¹⁰ or SO₂NR⁹R¹⁰;
R³ is independently selected from hydrogen, halo, C₁₋₄ alkyl, C₂₋₄ alkenyl, C₂₋₄ alkynyl, C₁₋₄ haloalkyl, (CH₂)ₘCN, S(O)ₙR⁶, C(O)R⁶;
R⁴ is independently selected from hydrogen, halo, C₁₋₄ alkyl, C₂₋₄ alkenyl, C₂₋₄ alkynyl, C₁₋₄ haloalkyl, (CH₂)ₘOR⁵, (CH₂)ₘCN, S(O)ₙR⁶, C(O)R⁶, phenyl or 5- or 6-membered heteroaryl, any of which phenyl or heteroaryl groups may be optionally substituted by halo, C₁₋₄ alkyl, C₁₋₄ haloalkyl, (CH₂)ₘCN or S(O)ₙR⁶;
or R² and an R³ group may form a fused 6-membered aryl or nitrogen containing heteroaryl ring, either of which may be optionally substituted by halo, C₁₋₄ alkyl, C₁₋₄ haloalkyl, (CH₂)ₘCN, (CH₂)ₘOR⁵, S(O)ₙR⁶, C(O)NR⁹R¹⁰ or SO₂NR⁹R¹⁰;
R⁵ is hydrogen, C₁₋₄ alkyl, C₁₋₄ haloalkyl, (CH₂)ₘphenyl or (CH₂)ₘNR⁷R⁸;
R⁶ is C₁₋₄ alkyl, optionally substituted by hydroxy;
R⁷ and R⁸ are independently selected from hydrogen and C₁₋₄ alkyl;
R⁹ and R¹⁰ are independently selected from hydrogen and C₁₋₄ alkyl, optionally substituted by hydroxy, or, taken together, R⁹ and R¹⁰ may form a 5- or 6-membered heterocyclic ring optionally substituted by hydroxy;
m is 0, 1, 2 or 3; and
n is 0, 1 or 2;
or a pharmaceutically acceptable salt thereof;
provided that when W is CR², V and X are CR³ and U and Y are CR⁴, then R¹ is not piperidin-4-yl substituted on nitrogen by phenyl or a 6-membered nitrogen containing heteroaryl.

3. A compound according to either claim 1 or 2, or a pharmaceutically acceptable salt thereof, wherein none of U, V, W, X and Y represent nitrogen.

4. A compound according to either claim 1 or 2, or a pharmaceutically acceptable salt thereof, wherein one of U, V, W, X and Y represent nitrogen.

5. A compound according to claim 4, or a pharmaceutically acceptable salt thereof, wherein X or Y represents nitrogen.

6. A compound according to any one of claims 1 to 5, or a pharmaceutically acceptable salt thereof, wherein R¹ represents phenyl, pyridyl or C₃₋₈ alkyl, optionally substituted by up to 3 groups selected from halo, C₁₋₄ alkyl, C₂₋₄ alkenyl, C₂₋₄ alkynyl, C₁₋₄ haloalkyl, (CH₂)ₘCN, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy groups.

7. A compound according to claim 6, or a pharmaceutically acceptable salt thereof, wherein R¹ represents phenyl, optionally substituted by up to 3 groups selected from halo, C₁₋₄ alkyl, C₂₋₄ alkenyl, C₂₋₄ alkynyl, C₁₋₄ haloalkyl, (CH₂)ₘCN, (CH₂)ₘOR⁵, (CH₂)ₘphenyl, 5- or 6-membered heteroaryl or 5- or 6-membered heterocyclyl, any of which substituent phenyl, heteroaryl or heterocyclyl groups may themselves be substituted by one or more C₁₋₄ alkoxy groups.

8. A compound according to any one of claims 1 to 7, or a pharmaceutically acceptable salt thereof, wherein R¹ is substituted by at least one group selected from C₁₋₄ alkyl, C₁₋₄ alkoxy, aryl, aryloxy, benzyloxy or trifluoromethoxy.

9. A compound according to any one of claims 1 to 8, or a pharmaceutically acceptable salt thereof, wherein R² represents phenyl or a 6-membered heteroaryl group, either of which may be optionally substituted by halo, C₁₋₄ alkyl, C₁₋₄ haloalkyl, (CH₂)ₘCN, S(O)ₙR⁶, C(O)NR⁹R¹⁰ or SO₂NR⁹R¹⁰.

10. A compound according to any one of claims 1 to 9, or a pharmaceutically acceptable salt thereof, wherein R¹ and R² are substituted by 1 or 2 groups.

11. A compound according to any one of claims 1 to 10, or a pharmaceutically acceptable salt thereof, wherein R² is substituted by (CH₂)ₘCN or S(O)ₙR⁶.

12. A compound according to any one of claims 1 to 11, or a pharmaceutically acceptable salt thereof, wherein R³ and R⁴ represent hydrogen, C₁₋₄ alkyl or halo.

13. A compound according to claim 12, or a pharmaceutically acceptable salt thereof, wherein R³ and R⁴ represent hydrogen.

14. A compound according to any one of claims 1 to 13, or a pharmaceutically acceptable salt thereof, wherein R⁵ represents C₁₋₄ alkyl, C₁₋₄ haloalkyl or (CH₂)ₘphenyl,

15. A compound according to any one of claims 1 to 14, or a pharmaceutically acceptable salt thereof, wherein R⁶ represents C₁₋₄ alkyl optionally substituted by hydroxy.

16. A compound according to any one of claims 1 to 8, 10, 11, 14 or 15, or a pharmaceutically acceptable salt thereof, wherein R² and an R³ group forms a fused 6-membered aryl or nitrogen containing heteroaryl ring optionally substituted by halo, C₁₋₄ alkyl, C₁₋₄ haloalkyl, (CH₂)ₘCN, (CH₂)ₘOR⁵ or S(O)ₙR⁶.

17. A compound according to claim 1 selected from:
3-(4-Methanesulfonylphenoxy)-1-(4-phenoxybenzyl)azetidine;
6-[1-(4-Phenoxybenzyl)azetidin-3-yloxy]naphthalene-2-carbonitrile;
3-(7-Methoxynaphthalen-2-yloxy)-1-(4-phenoxybenzyl)azetidine;
{4-[1-(4-Phenoxybenzyl)azetidin-3-yloxy]phenyl}acetonitrile;
3-[4-(2-Methoxyethyl)phenoxy]-1-(4-phenoxybenzyl)azetidine;
3-{4-[1-(4-Phenoxybenzyl)azetidin-3-yloxy]phenyl}propionitrile;
1-(4-Phenoxyhenzyl)-3-(3-phenoxyphenoxy)azetidine;
4-[1-(4-Phenoxybenzyl)azetidin-3-yloxy]benzonitrile;
4'-[1-(4-Phenoxybenzyl)azetidin-3-yloxy]biphenyl-4-carbonitrile;
3-(Biphenyl-3-yloxy)-1-(4-phenoxybenzyl)azetidine;
1-{4-[1-(4-Phenoxybenzyl)azetidin-3-yloxy]phenyl}ethanone;
2-Methyl-3-[1-(4-phenoxybenzyl)azetidin-3-yloxy]pyridine;
2'-Methyl-5-[1-(4-phenoxybenxyl)axetidin-3-yloxy]-[2,4']bipyridinyl;
3-Fluoro-4-[1-(4-phenoxybenzyl)azetidin-3-yloxy]benzonitrile;
3-(4'-Methanesulfonylbiphenyl-4-yloxy)-1-(4-phenoxybenzyl)azetidine;
5-Methanesulfonyl-2-{4-[1-{4-phenoxybenzyl)azelidin-3-yloxy]phenyl}pyridine;
2-Methanesulfonyl-5-{4-[1-(4-phenoxybenzyl)azetidin-3-yloxy]phenyl}pyridine,
4-{4-[1-(4-Phenoxybenzyl)azetidine-3-yloxy]phenyl}pyridine-2-carbonitrile;
2-Methyl-4-{4-[1-(4-phenoxybenzyl)azetidin-3-yloxy]phenyl}pyridine;
4'-[1-(4-Phenoxybenzyl)azetidin-3-yloxy]biphenyl-3-carbonitrile;
3-{6-[1-(4-Phenoxybenzyl)azetidin-3-yloxy]-pyridin-3-yl}benzonitrile;
3-{5-[1-(4-Phenoxybenzyl)azetidin-3-yloxy]pyridin-2-yl}benzonitrile;
2-(4-Methanesulfonylptieriyl)-5-[1-(4-phenoxybenzyl)azetidin-3-yloxy]pyridine;
6-[1-(4-Phenoxybenzyl)azetidin-3-yloxy]-[3,4']bipyridinyl-2'-carbonitrile;
5-(4-Methanesulfonylphenyl)-2-[1-(4-phenoxybenzyl)azetidin-3-yloxy]pyridine;
5-Methanesulfonyl-6'-[1-(4-phenoxybenzyl)azetidin-3-yloxy]-[2,3']bipyridinyl;
6'-Methanesulfonyl-6-[1-(4-phenoxybenzyl)azetidin-3-yloxy]-[3,3']bipyridinyl;
6'-Fluoro-6-[1-(4-phenoxybenzyl)azetidin-3-yloxy]-[3,3']bipyridinyl;
5-{4-[1-(2,2-Dimethylpropyl)azetidin-3-yloxy]phenyl}-2-methanesulfonylpyridine;
5-[4-(1-Benzylazetidin-3-yloxy)phenyl]-2-methanesulfonylpyridine;
2-Methanesulfonyl-5-{4-[1-(6-(4-methoxyphenyl)pyridin-3-ylmethyl)azetidin-3-yloxy]-phenyl}pyridine;
2-Methanesulfonyl-5-{4-[1-(3-phenoxybenzyl)azetidin-3-yloxy]phenyl}pyridine;
2-Methanesulfonyl-5-{4-[1-(3-methoxybenzyl)azetidin-3-yloxy]phenyl}pyridine;
5-{4-[1-(4-Benzyloxy-3-methoxybenzyl)azetidin-3-yloxy]phenyl}-2-methanesulfonylpyridine;
5-{4-[1-(3-Benzyloxybenzyl)azetidin-3-yloxy]-phenyl}-2-methanesulfonylpyridine;
2-Methanesulfonyl-5-{4-[1-(3-trifluoromethylbenzyl)azetidin-3-yloxy]phenyl} pyridine;
2-Methanesulfonyl-5-{4-[1-(3-methylbenzyl)axetidin-3-yloxy]phenyl}pyridine;
4-{3-[4-(6-Methanesulfonylpyridin-3-yl)phenoxy]azetidin-1-ylmethyl}benzonitrile;
2-Methanesulfonyl-5-{4-[1-(4-methoxybenzyl)azetidin-3-yloxy]phenyl}pyridine;
5-{4-[1-(4-Benzyloxybenzyl)azetidin-3-yloxy]phenyl}-2-methanesulfonylpyridine;
2-Methanesulfonyl-5-[4-(1-pyridin-2-ylmethylazetidin-3-yloxy)phenyl]pyridine;
2-Methansulfonyl-5-[4-(1-pyridin-3-ylmethylazetidin-3-yloxy)phenyl]pyridine;
3-{3-[4-(6-Methanesulfonylpyridin-3-yl)phenoxy]azetidin-1-ymethyl} quinoline;
4-(4-{3-[4-(6-Methanesulfonylpyridin-3-yl)phenoxy]azetidin-1-ymethyl}phenyl)morpholine;
5-[4-(1-Biphenyl-4-yl-methylazetidin-3-yloxy)phenyl]-2-methanesulfonylpyridine;
5-{4-[1-(4-Isopropylbenzyl)azetidin-3-yl-oxy]phenyl}-2-methanesulfonylpyridine;
[3-(4-{3-[4-(6-Methanesulfonylpyridin-3-yl)phenoxy]azetidin-1-ymethyl}phenoxy)-propyl]dimethylamine;
2-Methanesulfonyl-5-{4-[1-(4-propoxybenzyl)azetidin-3-yloxy]phenyl}pyridine;
2-Methanesulfonyl-5-{4-[1-(tetrahydropyran-4-ylmethyl)azetidin-3-yloxy]phenyl}pyridine;
2-Methanesulfonyl-5-{4-[1-(4-trifluoromethoxybenzyl)azetidin-3-yloxy]phenyl}pyridine;
5-{4-[1-(2,3-Dihydrobenzofuran-5-ylmethyl)azetidin-3-yloxy]phenyl}-2-methanesulfonylpyridine;
2-{3-[4-(6-Methanesulfonylpyridin-3-yl)phenoxy]azetidin-1-ymethyl}quinoline;
2-Methanesulfonyl-5-{4-[1-(6-methoxynaphtahalen-2-ylmethyl)azetidin-3-yloxy]-phenyl} pyridine;
5-{4-[1-(4-Imidazol-1-ylbenzyl)azetidin-3-yloxy]phenyl}-2-methanesulfonylpyridine;
5-{4-[1-(4-Fluoro-3-phenoxybenzyl)azetidin-3-yloxy]phenyl}-2-methanesulfonylpyridine;
2-Methanesulfonyl-5-{4-[1-(4-pyridin-2-ylbenzyl)azetidin-3-yloxy]phenyl}pyridine;
2-Fluoro-5-{3-[4-(6-methanesulfonylpyridin-3-yl)phenoxy]azetidin-1-ylmethyl}benzonitrile;
2-Methanesulfonyl-5-{4-[1-(6-methoxypyridin-3-ylmethyl)azetidin-3-yloxy]phenyl}pyridine;
5-(4-{3-[4-(6-Methanesulfonylpyridin-3-yl)phenoxy]azetidin-1-ymethyl}phenyl)pyrimidine;
2-Methanesulfonyl-5-{4-[1-(6-phenoxypyridin-3-ylmethyl)azetidin-3-yloxy]phenyl}pyridine;
5-{4-[1-(3,3-Dimethylbutyl)azetidin-3-yloxy]phenyl}-2-methanesulfonylpyridine; and
5-{3-[4-(6-Methanesulfonylpyridin-3-yl)phenoxy]azetidin-1-ymethyl}pyrimidine;
or a pharmaceutically acceptable salt of any one thereof.

18. A compound according to claim 1 comprising 5-{4-[1-(4-isopropyl-benzyl)azetidin-3-yl-oxy]phenyl}-2-methane-sulfonylpyridine.

19. A compound according to claim 1 comprising 2-Methanesulfonyl-5-{4-[1-(4-trifluoromethoxy-benzyl)azetidin-3-yloxy]phenyl} pyridine.

20. A pharmaceutical composition comprising a compound according to any one of claims 1 to 19, or a pharmaceutically acceptable salt thereof and a pharmaceutically acceptable carrier.

21. A compound according to any one claims 1 to 19, or a pharmaceutically acceptable salt thereof, for use in the treatment of a disease or condition in which GPR119 plays a role.

22. A compound according to any one of claims 1 to 19, or a pharmaceutically acceptable salt thereof, for use in the regulation of satiety or the treatment of obesity.

23. A compound according to any one of claims 1 to 19, or a pharmaceutically acceptable salt thereof, for use in the treatment of diabetes.

24. A compound according to any one of claims 1 to 19, or a pharmaceutically acceptable salt thereof, for use in the treatment of metabolic syndrome (syndrome X), impaired glucose tolerance, hyperlipidemia, hypertriglyceridemia, hypercholesterolemia, low HDL levels or hypertension.

## Patentansprüche

1. Verbindung der Formel (I): 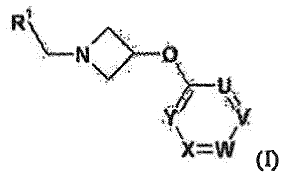 worin:
W CR² oder Stickstoff ist,
V und X jeweils unabhängig CR³ oder Stickstoff sind, U und Y jeweils unabhängig CR⁴ oder Stickstoff sind, mit der Maßgabe, dass nicht mehr als drei von U, V, W, X und Y Stickstoff sind,
R¹ Phenyl, Naphthyl, 6- bis 10-gliedriges Heteroaryl, 6-gliedriges Heterocyclyl, C₃₋₈-Cycloalkyl, 2,3-Dihydrobenzofuryl oder C₃₋₈-Alkyl ist, wobei jedes davon gegebenenfalls durch bis zu 3 Gruppen substituiert sein kann, ausgewählt aus Halogen, C₁₋₄-Alkyl, C₂₋₄-Alkenyl, C₂₋₄-Alkinyl, C₁₋₄-Halogenalkyl, (CH₂)ₘCN, C(O)NR⁹R¹⁰, C(O)R⁶, S(O)ₙR⁶, SO₂NR⁹R¹⁰, (CH₂)ₘOR⁵, (CH₂)ₘphenyl, 5- oder 6-gliedrigem Heteroaryl oder 5- oder 6-gliedrigem Heterocyclyl, wobei jede der Phenyl-, Heteroaryl- oder Heterocyclyl-Substituentengruppen selbst durch eine oder mehrere C₁₋₄-Alkoxy-, Halogen-, Cyano-, C₁₋₄-Alkyl-, C₁₋₄-Halogenalkyl-, (CH₂)ₘCN-, C(O)NR⁹R¹⁰-, C(O)R⁶-, S(O)ₙR⁶-, SO₂NR⁹R¹⁰-, NR¹¹C(O)NR⁹R¹⁰- oder (CH₂)ₘOR⁵-Gruppe(n) substituiert sein kann,
R², R³ und R⁴ unabhängig aus Wasserstoff, Halogen, C₁₋₄-Alkyl, C₂₋₄-Alkenyl, C₂₋₄-Alkinyl, C₁₋₄-Halogenalkyl, (CH₂)ₘOR⁵, (CH₂)ₘCN, S(O)ₙR⁶, C(O)NR⁹R¹⁰, S(O)₂NR⁹R¹⁰, NR¹¹C(O)NR⁹R¹⁰, C(O)R⁶, Phenyl oder 5- oder 6-gliedrigem Heteroaryl ausgewählt sind, wobei jede der Phenyl- oder Heteroarylgruppen gegebenenfalls durch Halogen, C₁₋₄-Alkyl, C₁₋₄-Alkoxy, Hydroxy, C₁₋₄-Halogenalkyl, (CH₂)ₘCN, S(O)ₙR⁶, C(O)NR⁹R¹⁰, C(O)R⁶, NR¹¹C(O)NR⁹R¹⁰ oder SO₂NR⁹R¹⁰ substituiert sein kann,
oder R² und eine R³-Gruppe oder R³ und eine benachbarte R⁴-Gruppe einen anellierten 6-gliedrigen Arylring oder Stickstoff-enthaltenden Heteroarylring bilden können, wobei jeder davon gegebenenfalls durch Halogen, C₁₋₄-Alkyl, C₁₋₄-Halogenalkyl, (CH₂)ₘCN, (CH₂)ₘOR⁵, S(O)ₙR⁶, C(O)R⁶, C(O)NR⁹R¹⁰ oder SO₂NR⁹R¹⁰ substituiert sein kann,
R⁵ Wasserstoff, C₁₋₄-Alkyl, C₁₋₄-Halogenalkyl, (CH₂)ₘNR⁷R⁸ oder (CH₂)ₘphenyl ist, wobei die Phenylgruppe gegebenenfalls durch Halogen, C₁₋₄-Alkyl, C₁₋₄-Alkoxy, C₁₋₄-Halogenalkyl oder CN substituiert sein kann,
R⁶ C₁₋₄-Alkyl ist, das gegebenenfalls durch Hydroxy oder NR⁷R⁸ substituiert ist,
R⁷ und R⁸ unabhängig aus Wasserstoff und C₁₋₄-Alkyl ausgewählt sind oder R⁷ und R⁸ einen 5- bis 7-gliedrigen heterocyclischen Ring bilden können, der gegebenenfalls durch Hydroxy oder Methyl substituiert ist,
R⁹ und R¹⁰ unabhängig aus Wasserstoff und C₁₋₄-Alkyl, das gegebenenfalls durch Hydroxy oder NR⁷R⁸ substituiert ist, ausgewählt sind, oder R⁹ und R¹⁰ zusammen einen 5- oder 6-gliedrigen heterocyclischen Ring bilden können, der gegebenenfalls durch Hydroxy oder C₁₋₄-Alkyl substituiert ist,
R¹¹ Wasserstoff oder Methyl ist,
m 0, 1, 2 oder 3 ist und
n 0, 1 oder 2 ist,
oder ein pharmazeutisch verträgliches Salz davon,
mit der Maßgabe, dass dann, wenn W CR² ist, V und X CR³ sind und U und Y CR⁴ sind, R¹ nicht Piperidin-4-yl ist, das am Stickstoff durch Phenyl oder ein 6-gliedriges Stickstoff-enthaltendes Heteroaryl substituiert ist.

2. Verbindung nach Anspruch 1, die eine Verbindung der Formel (III) ist: 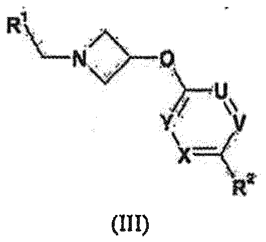 worin:
V und X jeweils unabhängig CR³ oder Stickstoff sind, U und Y jeweils unabhängig CR⁴ oder Stickstoff sind, mit der Maßgabe, dass nicht mehr als drei von U, V, X und Y Stickstoff sind,
R¹ Phenyl, Naphthyl, 6- bis 10-gliedriges Heteroaryl, 6-gliedriges Heterocyclyl, C₃₋₈-Cycloalkyl, 2,3-Dihydrobenzofuryl oder C₃-₈-Alkyl ist, wobei jedes davon gegebenenfalls durch bis zu 3 Gruppen substituiert sein kann, ausgewählt aus Halogen, C₁₋₄-Alkyl, C₂₋₄-Alkenyl, C₂-₄-Alkinyl, C₁₋₄-Halogenalkyl, (CH₂)ₘCN, (CH₂)ₘOR⁵, (CH₂)ₘphenyl, 5- oder 6-gliedrigem Heteroaryl oder 5- oder 6-gliedrigem Heterocyclyl, wobei jede der Phenyl-, Heteroaryl- oder Heterocyclyl-Substituentengruppen selbst durch eine oder mehrere C₁₋₄-Alkoxy-Gruppe(n) substituiert sein kann,
R² aus Halogen, C₁₋₄-Alkyl, C₂₋₄-Alkenyl, C₂₋₄-Alkinyl, C₁₋₄-Halogenalkyl, (CH₂)ₘOR⁵, (CH₂)ₘCN, S(O)ₙR⁶, C(O)R⁶, Phenyl oder 5- oder 6-gliedrigem Heteroaryl ausgewählt ist, wobei jede der Phenyl- oder Heteroarylgruppen gegebenenfalls durch Halogen, C₁₋₄-Alkyl, C₁₋₄-Halogenalkyl, (CH₂)ₘCN, S(O)ₙR⁶, C(O)NR⁹R¹⁰ oder SO₂NR⁹R¹⁰ substituiert sein kann,
R³ unabhängig aus Wasserstoff, Halogen, C₁₋₄-Alkyl, C₂₋₄-Alkenyl, C₂₋₄-Alkinyl, C₁₋₄-Halogenalkyl, (CH₂)ₘCN, S(O)ₙR⁶, C(O)R⁶ ausgewählt ist,
R⁴ unabhängig aus Wasserstoff, Halogen, C₁₋₄-Alkyl, C₂-₄-Alkenyl, C₂-₄-Alkinyl, C₁₋₄-Halogenalkyl, (CH₂)ₘOR⁵, (CH₂)ₘCN, S(O)ₙR⁶, C(O)R⁶, Phenyl oder 5- oder 6-gliedrigem Heteroaryl ausgewählt ist, wobei jede der Phenyl- oder Heteroarylgruppen gegebenenfalls durch Halogen, C₁₋₄-Alkyl, C₁₋₄-Halogenalkyl, (CH₂)ₘCN oder S(O)ₙR⁶ substituiert sein kann,
oder R² und eine R³-Gruppe einen anellierten 6-gliedrigen Arylring oder Stickstoff-enthaltenden Heteroarylring bilden können, wobei jeder davon gegebenenfalls durch Halogen, C₁₋₄-Alkyl, C₁₋₄-Halogenalkyl, (CH₂)ₘCN, (CH₂)ₘOR⁵, S(O)ₙR⁶, C(O)NR⁹R¹⁰ oder SO₂NR⁹R¹⁰ substituiert sein kann,
R⁵ Wasserstoff, C₁₋₄-Alkyl, C₁₋₄-Halogenalkyl, (CH₂)ₘphenyl oder (CH₂)ₘNR⁷R⁸ ist,
R⁶ C₁₋₄-Alkyl ist, das gegebenenfalls durch Hydroxy substituiert ist,
R⁷ und R⁸ unabhängig aus Wasserstoff und C₁₋₄-Alkyl ausgewählt sind,
R⁹ und R¹⁰ unabhängig aus Wasserstoff und C₁₋₄-Alkyl, das gegebenenfalls durch Hydroxy substituiert ist, ausgewählt sind, oder R⁹ und R¹⁰ zusammen einen 5- oder 6-gliedrigen heterocyclischen Ring bilden können, der gegebenenfalls durch Hydroxy substituiert ist,
m 0, 1, 2 oder 3 ist und
n 0, 1 oder 2 ist,
oder ein pharmazeutisch verträgliches Salz davon,
mit der Maßgabe, dass dann, wenn W CR² ist, V und X CR³ sind und U und Y CR⁴ sind, R¹ nicht Piperidin-4-yl ist, das am Stickstoff durch Phenyl oder ein 6-gliedriges Stickstoff-enthaltendes Heteroaryl substituiert ist.

3. Verbindung nach Anspruch 1 oder 2 oder ein pharmazeutisch verträgliches Salz davon, wobei keine von U, V, W, X und Y Stickstoff darstellt.

4. Verbindung nach Anspruch 1 oder 2 oder ein pharmazeutisch verträgliches Salz davon, wobei eine von U, V, W, X und Y Stickstoff darstellt.

5. Verbindung nach Anspruch 4 oder ein pharmazeutisch verträgliches Salz davon, wobei X oder Y Stickstoff darstellt.

6. Verbindung nach einem der Ansprüche 1 bis 5 oder ein pharmazeutisch verträgliches Salz davon, wobei R¹ Phenyl, Pyridyl oder C₃₋₈-Alkyl darstellt, gegebenenfalls substituiert durch bis zu 3 Gruppen, ausgewählt aus Halogen, C₁₋₄-Alkyl, C₂₋₄-Alkenyl, C₂₋₄-Alkinyl, C₁₋₄-Halogenalkyl, (CH₂)ₘCN, (CH₂)ₘOR⁵, (CH₂)ₘphenyl, 5- oder 6-gliedrigem Heteroaryl oder 5- oder 6-gliedrigem Heterocyclyl, wobei jede der Phenyl-, Heteroaryl- oder Heterocyclyl-Substituentengruppen selbst durch eine oder mehrere C₁₋₄-Alkoxy-Gruppe(n) substituiert sein kann.

7. Verbindung nach Anspruch 6 oder ein pharmazeutisch verträgliches Salz davon, wobei R¹ Phenyl darstellt, gegebenenfalls substituiert durch bis zu 3 Gruppen, ausgewählt aus Halogen, C₁₋₄-Alkyl, C₂-₄-Alkenyl, C₂-₄-Alkinyl, C₁₋₄-Halogenalkyl, (CH₂)ₘCN, (CH₂)ₘOR⁵, (CH₂)ₘphenyl, 5- oder 6-gliedrigem Heteroaryl oder 5- oder 6-gliedrigem Heterocyclyl, wobei jede der Phenyl-, Heteroaryl- oder Heterocyclyl-Substituentengruppen selbst durch eine oder mehrere C₁₋₄-Alkoxy-Gruppe(n) substituiert sein kann.

8. Verbindung nach einem der Ansprüche 1 bis 7 oder ein pharmazeutisch verträgliches Salz davon, wobei R¹ durch mindestens eine Gruppe substituiert ist, die aus C₁₋₄-Alkyl, C₁-₄-Alkoxy, Aryl, Aryloxy, Benzyloxy oder Trifluormethoxy ausgewählt ist.

9. Verbindung nach einem der Ansprüche 1 bis 8 oder ein pharmazeutisch verträgliches Salz davon, wobei R² Phenyl oder eine 6-gliedrige Heteroarylgruppe darstellt, die jeweils gegebenenfalls durch Halogen, C₁₋₄-Alkyl, C₁₋₄-Halogenalkyl, (CH₂)ₘCN, S(O)ₙR⁶, C(O)NR⁹R¹⁰ oder SO₂NR⁹R¹⁰ substituiert sein kann.

10. Verbindung nach einem der Ansprüche 1 bis 9 oder ein pharmazeutisch verträgliches Salz davon, wobei R¹ und R² durch 1 oder 2 Gruppe(n) substituiert sind.

11. Verbindung nach einem der Ansprüche 1 bis 10 oder ein pharmazeutisch verträgliches Salz davon, wobei R² durch (CH₂)ₘCN oder S(O)ₙR⁶ substituiert ist.

12. Verbindung nach einem der Ansprüche 1 bis 11 oder ein pharmazeutisch verträgliches Salz davon, wobei R³ und R⁴ Wasserstoff, C₁₋₄-Alkyl oder Halogen darstellen.

13. Verbindung nach Anspruch 12 oder ein pharmazeutisch verträgliches Salz davon, wobei R³ und R⁴ Wasserstoff darstellen.

14. Verbindung nach einem der Ansprüche 1 bis 13 oder ein pharmazeutisch verträgliches Salz davon, wobei R⁵ C₁₋₄-Alkyl, C₁₋₄-Halogenalkyl oder (CH₂)ₘphenyl darstellt.

15. Verbindung nach einem der Ansprüche 1 bis 14 oder ein pharmazeutisch verträgliches Salz davon, wobei R⁶ C₁₋₄-Alkyl darstellt, das gegebenenfalls durch Hydroxy substituiert ist.

16. Verbindung nach einem der Ansprüche 1 bis 8, 10, 11, 14 oder 15 oder ein pharmazeutisch verträgliches Salz davon, wobei R² und eine R³-Gruppe einen anellierten 6-gliedrigen Arylring oder Stickstoff-enthaltenden Heteroarylring bilden, der gegebenenfalls durch Halogen, C₁₋₄-Alkyl, C₁₋₄-Halogenalkyl, (CH₂)ₘCN, (CH₂)ₘOR⁵ oder S(O)ₙR⁶ substituiert ist.

17. Verbindung nach Anspruch 1, ausgewählt aus:
3-(4-Methansulfonylphenoxy)-1-(4-phenoxybenzyl)azetidin,
6-[1-(4-Phenoxybenzyl)azetidin-3-yloxy]naphthalin-2-carbonitril,
3-(7-Methoxynaphthalin-2-yloxy)-1-(4-phenoxybenzyl)azetidin,
{4-[1-(4-Phenoxybenzyl)azetidin-3-yloxy]phenyl}acetonltril,
3-[4-(2-Methoxyethyl)phenoxy]-1-(4-phenoxybenzyl)azetidin,
3-{4-[1-(4-Phenoxybenzyl)azetidin-3-yloxy]phenyl}propionitril,
1-(4-Phenoxybenzyl)-3-(3-phenoxyphenoxy)azetidin,
4-[1-(4-Phenoxybenzyl)azetidin-3-yloxy]benzonitril,
4'-[1-(4-Phenoxybenzyl)azetidin-3-yloxy]biphenyl-4-carbonitril,
3-(Biphenyl-3-yloxy)-1-(4-phenoxybenzyl)azetidin,
1-{4-[1-(4-Phenoxybenzyl)azetidi n-3-yloxy] phenyl}ethanon,
2-Methyl-3-[1-(4-phenoxybenzyl)azetidin-3-yloxy]pyridin,
2'-Methyl-5-[1-(4-phenoxybenzyl)azetidin-3-yloxy]-[2,4']bipyridinyl,
3-Fluor-4-[1-(4-phenoxybenzyl)azetidin-3-yloxy]benzonitril,
3-(4'-Methansulfonylbiphenyl-4-yloxy)-1-(4-phenoxybenzyl)azetidin,
5-Methansulfonyl-2-{4-[1-(4-phenoxybenzyl)azetidin-3-yloxy]phenyl}pyridin,
2-Methansulfonyl-5-{4-[1-(4-phenoxybenzyl)azetidin-3-yloxy]phenyl}pyridin,
4-{4-[1-(4-Phenoxybenzyl)azetidin-3-yloxy]phenyl}pyridin-2-carbonitril,
2-Methyl-4-{4-[1-(4-phenoxybenzyl)azetidin-3-yloxy]phenyl}pyridin,
4'-[1-(4-Phenoxybenzyl)azetidin-3-yloxy]biphenyl-3-carbonitril,
3-{6-[1-(4-Phenoxybenzyl)azetidin-3-yloxy]-pyridin-3-yl}benzonitril,
3-{5-[1-(4-Phenoxybenzyl)azetidin-3-yloxy]-pyridin-2-yl}benzonitril,
2-(4-Methansulfonylphenyl)-5-[1-(4-phenoxybenzyl)azetidin-3-yloxy]pyridin,
6-[1-(4-Phenoxybenzyl)azetidin-3-yloxy]-[3,4']bipyridinyl-2'-carbonitril,
5-(4-Methansulfonylphenyl)-2-[1-(4-phenoxybenzyl)azetidin-3-yloxy]pyridin,
5-Methansulfonyl-6'-[1-(4-phenoxybenzyl)azetidin-3-yloxy]-[2,3']bipyridinyl,
6'-Methansulfonyl-6-[1-(4-phenoxybenzyl)azetidin-3-yloxy]-[3,3']bipyridinyl,
6'-Fluor-6-[1-(4-phenoxybenzyl)azetidin-3-yloxy]-[3,3']bipyridinyl,
5-{4-[1-(2,2-Dimethylpropyl)azetidin-3-yloxy]phenyl}-2-methansulfonylpyridin,
5-[4-(1-Benzylazetidin-3-yloxy)phenyl]-2-methansulfonylpyridin,
2-Methansulfonyl-5-{4-[1-(6-(4-methoxyphenyl)pyridin-3-ylmethyl)azetidin-3-yloxy]phenyl}pyridin,
2-Methansulfonyl-5-{4-[1-(3-phenoxybenzyl)azetidin-3-yloxy]phenyl}pyridin,
2-Methansulfonyl-5-{4-[1-(3-methoxybenzyl)azetidin-3-yloxy]phenyl}pyridin,
5-{4-[1-(4-Benzyloxy-3-methoxybenzyl)azetidin-3-yloxy]phenyl}-2-methansulfonyl-pyridin,
5-{4-[1-(3-Benzyloxybenzyl)azetidin-3-yloxy]-phenyl}-2-methansulfonylpyridin,
2-Methansulfonyl-5-{4-[1-(3-trifluormethylbenzyl)azetidin-3-yloxy]phenyl}pyridin,
2-Methansulfonyl-5-{4-[1-(3-methylbenzyl)azetidin-3-yloxy]phenyl}pyridin,
4-{3-[4-(6-Methansulfonylpyridin-3-yl)phenoxy]azetidin-1-ylmethyl}benzonitril,
2-Methansulfonyl-5-{4-[1-(4-methoxybenzyl)azetidin-3-yloxy]phenyl}pyridin,
5-{4-[1-(4-Benzyloxybenzyl)azetidin-3-yloxy]phenyl}-2-methansulfonylpyridin,
2-Methansulfonyl-5-[4-(1-pyridin-2-ylmethylazetidin-3-yloxy)phenyl]pyridin,
2-Methansulfonyl-5-[4-(1-pyridin-3-ylmethylazetidin-3-yloxy)phenyl]pyridin,
3-{3-[4-(6-Methansulfonylpyridin-3-yl)phenoxy]azetidin-1-ylmethyl}chinolin,
4-(4-{3-[4-(6-Methansulfonylpyridin-3-yl)phenoxy]azetidin-1-ylmethyl}phenyl)-morpholin,
5-[4-(1-Biphenyl-4-yl-methylazetidin-3-yloxy)phenyl]-2-methansulfonylpyridin,
5-{4-[1-(4-Isopropylbenzyl)azetidin-3-yloxy]phenyl}-2-methansulfonylpyridin,
[3-(4-{3-[4-(6-Methansulfonylpyridin-3-yl)phenoxy]azetidin-1-ylmethyl}phenoxy)-propyl]dimethylamin,
2-Methansulfonyl-5-{4-[1-(4-propoxybenzyl)azetidin-3-yloxy]phenyl}pyridin,
2-Methansulfonyl-5-{4-[1-(tetrahydropyran-4-ylmethyl)azetidin-3-yloxy]phenyl}pyridin,
2-Methansulfonyl-5-{4-[1-(4-trifluormethoxybenzyl)azetidin-3-yloxy]phenyl}pyridin,
5-{4-[1-(2,3-Dihydrobenzofuran-5-ylmethyl)azetidin-3-yloxy]phenyl}-2-methansulfonylpyridin,
2-{3-[4-(6-Methansulfonylpyridin-3-yl)phenoxy]azetidin-1-ylmethyl}chinolin,
2-Methansulfonyl-5-{4-[1-(6-methoxynaphthalin-2-ylmethyl)azetidin-3-yloxy]phenyl}pyridin,
5-{4-[1-(4-Imidazol-1-ylbenzyl)azetidin-3-yloxy]phenyl}-2-methansulfonylpyridin,
5-{4-[1-(4-Fluor-3-phenoxybenzyl)azetidin-3-yloxy]phenyl}-2-methansulfonylpyridin,
2-Methansulfonyl-5-{4-[1-(4-pyridin-2-ylbenzyl)azetidin-3-yloxy]phenyl}pyridin,
2-Fluor-5-{3-[4-(6-Methansulfonylpyridin-3-yl)phenoxy]azetidin-1-ylmethyl}benzonitril,
2-Methansulfonyl-5-{4-[1-(6-methoxypyridin-3-ylmethyl)azetidin-3-yloxy]phenyl}-pyridin,
5-(4-{3-[4-(6-Methansulfonylpyridin-3-yl)phenoxy]azetidin-1-ylmethyl}phenyl)-pyrimidin,
2-Methansulfonyl-5-{4-[1-(6-phenoxypyridin-3-ylmethyl)azetidin-3-yloxy]phenyl}-pyridin,
5-{4-[1-(3,3-Dimethylbutyl)azetidin-3-yloxy]phenyl}-2-methansulfonylpyridin und
5-{3-[4-(6-Methansulfonylpyridin-3-yl)phenoxy]azetidin-1-ylmethyl}pyrimidin
oder ein pharmazeutisch verträgliches Salz davon.

18. Verbindung nach Anspruch 1, umfassend 5-{4-[1-(4-Isopropylbenzyl)azetidin-3-yloxy]-phenyl}-2-methansulfonylpyridin.

19. Verbindung nach Anspruch 1, umfassend 2-Methansulfonyl-5-{4-[1-(4-trifluormethoxy-benzyl)azetidin-3-yloxy]phenyl}pyridin.

20. Pharmazeutische Zusammensetzung, die eine Verbindung nach einem der Ansprüche 1 bis 19 oder ein pharmazeutisch verträgliches Salz davon und einen pharmazeutisch verträglichen Träger umfasst.

21. Verbindung nach einem der Ansprüche 1 bis 19 oder ein pharmazeutisch verträgliches Salz davon zur Verwendung bei der Behandlung einer Erkrankung oder eines Zustands, in dem GPR119 eine Rolle spielt.

22. Verbindung nach einem der Ansprüche 1 bis 19 oder ein pharmazeutisch verträgliches Salz davon zur Verwendung bei der Regulierung der Sattheit oder der Behandlung von Fettsucht.

23. Verbindung nach einem der Ansprüche 1 bis 19 oder ein pharmazeutisch verträgliches Salz davon zur Verwendung bei der Behandlung von Diabetes.

24. Verbindung nach einem der Ansprüche 1 bis 19 oder ein pharmazeutisch verträgliches Salz davon zur Verwendung bei der Behandlung von metabolischem Syndrom (Syndrom X), beeinträchtigter Glukosetoleranz, Hyperlipidämie, Hypertriglyceridämie, Hyperchol-esterinämie, niedrigen HDL-Konzentrationen oder Hypertonie.

## Revendications

1. Composé de formule (I) 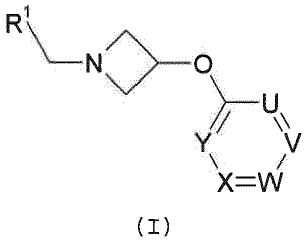 dans laquelle :
W est CR² ou l'azote ;
chacun de V et X est indépendamment CR³ ou l'azote ;
chacun de U et Y est indépendamment CR⁴ ou l'azote, à la condition qu'au plus trois parmi U, V, W, X et Y soient l'azote ;
R¹ est phényle, naphtyle, hétéroaryle de 6 à 10 chaînons, hétérocyclyle à 6 chaînons, cycloalkyle en C₃ à C₈, 2,3-dihydrobenzofuryle, ou alkyle en C₃ à C₈ ; l'un quelconque de ceux-ci pouvant être éventuellement substitué par jusqu'à 3 groupes choisis parmi halogéno, alkyle en C₁ à C₄, alcényle en C₂ à C₄, alcynyle en C₂ à C₄, halogénoalkyle en C₁ à C₄, (CH₂)ₘCN, C(O)NR⁹R¹⁰, C(O)R⁶, S(O)ₙR⁶, SO₂NR⁹R¹⁰, (CH₂)ₘOR⁵, (CH₂)ₘ-phényle, hétéroaryle à 5 ou 6 chaînons ou hétérocyclyle à 5 ou 6 chaînons, l'un quelconque des groupes substituants phényle, hétéroaryle ou hétérocyclyle pouvant lui-même être substitué par un ou plusieurs groupes alcoxy en C₁ à C₄, halogéno, cyano, alkyle en C₁ à C₄ , halogénoalkyle en C₁ à C₄, (CH₂)ₘCN, C (O) NR⁹R¹⁰, C(O)R⁶, S(O)ₙR⁶, SO₂NR⁹R¹⁰, NR¹¹C(O)NR⁹R¹⁰ ou (CH₂)ₘOR⁵ ;
R², R³ et R⁴ sont indépendamment choisis parmi l'hydrogène, halogéno, alkyle en C₁ à C₄, alcényle en C₂ à C₄, alcynyle en C₂ à C₄, halogénoalkyle en C₁ à C₄, (CH₂)ₘOR⁵, (CH₂)ₘCN, S(O)ₙR⁶, C(O)NR⁹R¹⁰, S(O)₂NR⁹R¹⁰, NR¹¹C(O) NR⁹R¹⁰, C(O)R⁶, phényle ou hétéroaryle à 5 ou 6 chaînons, l'un quelconque des groupes phényle ou hétéroaryle pouvant être éventuellement substitué par halogéno, alkyle en C₁ à C₄, alcoxy en C₁ à C₄, hydroxy, halogénoalkyle en C₁ à C₄, (CH₂)ₘCN, S(O)ₙR⁶, C(O)NR⁹R¹⁰, C(O)R⁶, NR¹¹C(O)NR⁹R¹⁰ ou SO₂NR⁹R¹⁰ _{;}
ou bien R² et un groupe R³, ou R³ et un groupe R⁴ adjacent, peuvent former un aryle condensé à 6 chaînons ou un cycle hétéroaryle azoté, l'un ou l'autre pouvant être éventuellement substitué par halogéno, alkyle en C₁ à C₄, halogénoalkyle en C₁ à C₄, (CH₂)ₘCN, (CH₂)ₘOR⁵, S(O)ₙR⁶, C(O)R⁶, C(O)NR⁹R¹⁰ ou SO₂NR⁹R¹⁰ ;
R⁵ est l'hydrogène, alkyle en C₁ à C₄, halogénoalkyle en C1 à C₄ , (CH₂)ₘNR⁷R⁸, ou (CH₂)ₘ-phényle, dont le groupe phényle peut être éventuellement substitué par halogéno, alkyle en C₁ à C₄, alcoxy en C₁ à C₄, halogénoalkyle en C₁ à C₄ ou CN ;
R⁶ est un alkyle en C₁ à C₄ éventuellement substitué par hydroxy ou NR⁷R⁸ ;
R⁷ et R⁸ sont indépendamment choisis parmi l'hydrogène et alkyle en C₁ à C₄, ou bien R⁷ et R⁸ peuvent former un hétérocycle de 5 à 7 chaînons éventuellement substitué par hydroxy ou méthyle ;
R⁹ et R¹⁰ sont indépendamment choisis parmi l'hydrogène et alkyle en C₁ à C₄ éventuellement substitué par hydroxy ou NR⁷R⁸ ou bien, pris ensemble, R⁹ et R¹⁰ peuvent former un hétérocycle à 5 ou 6 chaînons éventuellement substitué par hydroxy ou alkyle en C₁ à C₄
R¹¹ est l'hydrogène ou méthyle ;
m est 0, 1, 2 ou 3 ; et
n est 0, 1 ou 2 ;
ou un sel pharmaceutiquement acceptable de celui-cri à la condition que, lorsque W est CR², V et X sont CR³ et U et Y sont CR⁴, alors R¹ ne soit pas pipéridin-4-yle substitué sur l'azote par phényle ou un hétéroaryle azoté à 6 chaînons.

2. Composé selon la revendication 1, qui est un composé de formule (III) : 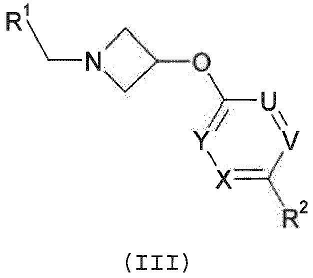 dans laquelle :
chacun de V et X est indépendamment CR³ ou l'azote ;
chacun de U et Y est indépendamment CR⁴ ou l'azote, à la condition qu'au plus trois parmi U, V, X et Y soient l'azote ;
R¹ est phényle, naphtyle, hétéroaryle de 6 à 10 chaînons, hétérocyclyle à 6 chaînons, cycloalkyle en C₃ à C₈, 2,3-dihydrobenzofuryle, ou alkyle en C₃ à C₈ ; l'un quelconque de ceux-ci pouvant être éventuellement substitué par jusqu'à 3 groupes choisis parmi halogéno, alkyle en C₁ à C₄, alcényle en C₂ à C₄, alcynyle en C₂ à C₄, halogénoalkyle en C₁ à C₄ , (CH₂)ₘCN, (CH₂)ₘOR⁵, (CH₂)ₘ-phényle, hétéroaryle à 5 ou 6 chaînons ou hétérocyclyle à 5 ou 6 chaînons, l'un quelconque des groupes substituants phényle, hétéroaryle et hétérocyclyle pouvant lui-même être substitué par un ou plusieurs groupes alcoxy en C₁ à C₄ ;
R² est choisi parmi halogéno, alkyle en C₁ à C₄, alcényle en C₂ à C₄ , alcynyle en C₂ à C₄, halogénoalkyle en C₁ à C₄, (CH₂)ₘOR⁵, (CH₂)ₘCN, S(O)ₙR⁶ C(O)R⁶, phényle ou hétéroaryle à 5 ou 6 chaînons, l'un quelconque des groupes phényle ou hétéroaryle pouvant être éventuellement substitué par halogéno, alkyle en C₁ à C₄, halogénoalkyle en C₁ à C₄, (CH₂)ₘCN, S(O)ₙR⁶, C(O)NR⁹R¹⁰ ou SO₂NR⁹R¹⁰ ;
R³ est indépendamment choisi parmi l'hydrogène, halogéno, alkyle en C₁ à C₄, alcényle en C₂ à C₄, alcynyle en C₂ à C₄, halogénoalkyle en C₁ à C₄, (CH₂)ₘCN, S(O)ₙR⁶, C(O)R⁶ ;
R⁴ est indépendamment choisi parmi l'hydrogène, halogéno, alkyle en C₁ à C₄, alcényle en C₂ à C₄, alcynyle en C₂ à C₄, halogénoalkyle en C₁ à C₄, (CH₂)ₘOR⁵, (CH₂)ₘCN, S(O)ₙR⁶, C(O)R⁶, phényle ou hétéroaryle à 5 ou 6 chaînons, l'un quelconque des groupes phényle et hétéroaryle pouvant être éventuellement substitué par halogéno, alkyle en C₁ à C₄, halogénoalkyle en C₁ à C₄, (CH₂)ₘCN ou S(O)ₙR⁶ ;
ou bien R² et un groupe R³ peuvent former un aryle condensé à 6 chaînons ou un cycle hétéroaryle azoté, l'un ou l'autre pouvant être éventuellement substitué par halogéno, alkyle en C₁ à C₄, halogénoalkyle en C₁ à C₄, (CH₂)ₘCN, (CH₂)ₘOR⁵, S(O)ₙR⁶, C (O) NR⁹R¹⁰ ou SO₂NR⁹R¹⁰ _{;}
R⁵ est l'hydrogène, alkyle en C₁ à C₄, halogénoalkyle en C1 à C₄ , (CH₂)ₘ-phényle ou (CH₂)ₘNR⁷R⁸ ;
R⁶ est un alkyle en C₁ à C₄ éventuellement substitué par hydroxy ;
R⁷ et R⁸ sont indépendamment choisis parmi l'hydrogène et alkyle en C₁ à C₄ ;
R⁹ et R¹⁰ sont indépendamment choisis parmi l'hydrogène et alkyle en C₁ à C₄, éventuellement substitué par hydroxy ou bien, pris ensemble, R⁹ et R¹⁰ peuvent former un hétérocycle à 5 ou 6 chaînons éventuellement substitué par hydroxy ;
m est 0, 1, 2 ou 3 ; et
n est 0, 1 ou 2 ;
ou un sel pharmaceutiquement acceptable de celui-ci ;
à la condition que, lorsque W est CR², V et X sont CR³ et U et Y sont CR⁴, alors R¹ ne soit pas pipéridin-4-yle substitué sur l'azote par phényle ou un hétéroaryle azoté à 6 chaînons.

3. Composé selon l'une ou l'autre des revendications 1 et 2, ou sel pharmaceutiquement acceptable de celui-ci, dans lequel aucun parmi U, V, W, X et Y ne représente l'azote.

4. Composé selon l'une ou l'autre des revendications 1 et 2, ou sel pharmaceutiquement acceptable de celui-ci, dans lequel l'un parmi U, V, W, X et Y représente l'azote.

5. Composé selon la revendication 4, ou sel pharmaceutiquement acceptable de celui-ci, dans lequel X ou Y représente l'azote.

6. Composé selon l'une quelconque des revendications 1 à 5, ou sel pharmaceutiquement acceptable de celui-ci, dans lequel R¹ représente phényle, pyridyle ou alkyle en C₃ à C₈, éventuellement substitué par jusqu'à 3 groupes choisis parmi halogéno, alkyle en C₁ à C₄, alcényle en C₂ à C₄, alcynyle en C₂ à C₄, halogénoalkyle en C₁ à C₄, (CH₂)ₘCN, (CH₂)ₘOR⁵, (CH₂)ₘ-phényle, hétéroaryle à 5 ou 6 chaînons ou hétérocyclyle à 5 ou 6 chaînons, l'un quelconque des groupes substituants phényle, hétéroaryle et hétérocyclyle pouvant lui-même être substitué par un ou plusieurs groupes alcoxy en C₁ à C₄.

7. Composé selon la revendication 6, ou sel pharmaceutiquement acceptable de celui-ci, dans lequel R¹ représente phényle éventuellement substitué par jusqu'à 3 groupes choisis parmi halogéno, alkyle en C₁ à C₄, alcényle en C₂ à C₄, alcynyle en C₂ à C₄, halogénoalkyle en C₁ à C₄, (CH₂)ₘCN, (CH₂)ₘOR⁵, (CH₂)ₘ-phényle, hétéroaryle à 5 ou 6 chaînons ou hétérocyclyle à 5 ou 6 chaînons, l'un quelconque des groupes substituants phényle, hétéroaryle et hétérocyclyle pouvant lui-même être substitué par un ou plusieurs groupes alcoxy en C₁ à C₄.

8. Composé selon l'une quelconque des revendications 1 à 7, ou sel pharmaceutiquement acceptable de celui-ci, dans lequel R¹ est substitué par au moins un groupe choisi parmi alkyle en C₁ à C₄, alcoxy en C₁ à C₄, aryle, aryloxy, benzyloxy et trifluorométhoxy.

9. Composé selon l'une quelconque des revendications 1 à 8, ou sel pharmaceutiquement acceptable de celui-ci, dans lequel R² représente phényle ou un groupe hétéroaryle à 6 chaînons, l'un ou l'autre pouvant être éventuellement substitué par halogéno, alkyle en C₁ à C₄, halogénoalkyle en C₁ à C₄, (CH₂)ₘCN, S(O)ₙR⁶, C(O)NR⁹R¹⁰ ou SO₂NR⁹R¹⁰_{.}

10. Composé selon l'une quelconque des revendications 1 à 9, ou sel pharmaceutiquement acceptable de celui-ci, dans lequel R¹ et R² sont substitués par 1 ou 2 groupes.

11. Composé selon l'une quelconque des revendications 1 à 10, ou sel pharmaceutiquement acceptable de celui-ci, dans lequel R² est substitué par (CH₂)ₘCN ou S(O)ₙR⁶.

12. Composé selon l'une quelconque des revendications 1 à 11, ou sel pharmaceutiquement acceptable de celui-ci, dans lequel R³ et R⁴ représentent l'hydrogène, alkyle en C₁ à C₄ ou halogéno.

13. Composé selon la revendication 12 ou sel pharmaceutiquement acceptable de celui-ci, dans lequel R³ et R⁴ représentent l'hydrogène.

14. Composé selon l'une quelconque des revendications 1 à 13, ou sel pharmaceutiquement acceptable de celui-ci, dans lequel R⁵ représente alkyle en C₁ à C₄ , halogénoalkyle en C₁ à C₄ ou (CH₂)ₘ-phényle.

15. Composé selon l'une quelconque des revendications 1 à 14, ou sel pharmaceutiquement acceptable de celui-ci, dans lequel R⁶ représente un alkyle en C₁ à C₄ éventuellement substitué par hydroxy.

16. Composé selon l'une quelconque des revendications 1 à 8, 10, 11, 14 ou 15, ou sel pharmaceutiquement acceptable de celui-ci, dans lequel R² et un groupe R³ forment un aryle condensé à 6 chaînons ou un cycle hétéroaryle azoté éventuellement substitué par halogéno, alkyle en C₁ à C₄, halogénoalkyle en C₁ à C₄, (CH₂)ₘCN, (CH₂)ₘOR⁵ ou S(O)ₙR⁶.

17. Composé selon la revendication 1, choisi parmi les suivants :
3-(4-méthanesulfonylphénoxy)-1-(4-phénoxybenzyl)-azétidine ;
6-[1-(4-phénoxybenzyl)azétidin-3-yloxy]naphtalène-2-carbonitrile ;
3-(7-méthoxynaphtalén-2-yloxy)-1-(4-phénoxybenzyl)-azétidine ;
{4-[1-(4-phénoxybenzyl)azétidin-3-yloxy]phényl}-acétonitrile ;
3-[4-(2-méthoxyéthyl)phénoxy]-1-(4-phénoxybenzyl)-azétidine ;
3-{4-[1-(4-phénoxybenzyl)azétidin-3-yloxy]phényl}-propionitrile ;
1-(4-phénoxybenzyl)-3-(3-phénoxyphénoxy)azétidine ;
4-[1-(4-phénoxybenzyl)azétidin-3-yloxy]benzonitrile ;
4'-[1-(4-phénoxybenzyl)azétidin-3-yloxy]biphényl-4-carbonitrile ;
3-(biphényl-3-yloxy)-1-(4-phénoxybenzyl)azétidine ;
1-{4-[1-(4-phénoxybenzyl)azétidin-3-yloxy]phényl}-éthanone ;
2-méthyl-3-[1-(4-phénoxybenzyl)azétidin-3-yloxyl-pyridine ;
2'-méthyl-5-[1-(4-phénoxybenzyl)azétidin-3-yloxy]-[2,4']bipyridinyle ;
3-fluoro-4-[1-(4-phénoxybenzyl)azétidin-3-yloxy]-benzonitrile ;
3-(4'-méthanesulfonylbiphényl-4-yloxy)-1-(4-phénoxybenzyl)azétidine ;
5-méthanesulfonyl-2-{4-[1-(4-phénoxybenzyl)azétidin-3-yloxy]phényl}pyridine ;
2-méthanesulfonyl-5-{4-[1-(4-phénoxybenzyl)azétidin-3-yloxy]phényl}pyridine ;
4-{4-[1-(4-phénoxybenzyl)azétidin-3-yloxy]phényl}-pyridine-2-carbonitrile ;
2-méthyl-4-{4-[1-(4-méthoxybenzyl)azétidin-3-yloxy]-phényl}pyridine ;
4'-[1-(4-phénoxybenzyl)azétidin-3-yloxy]biphényl-3-carbonitrile ;
3-{6-[1-(4-phénoxybenzyl)azétidin-3-yloxy]pyridin-3-yl}benzonitrile ;
3-{5-[1-(4-phénoxybenzyl)azétidin-3-yloxy]pyridin-2-yl}benzonitrile ;
2-(4-méthanesulfonylphényl)-5-[1-(4-phénoxybenzyl)-azétidin-3-yloxylpyridine ;
6-[1-(4-phénoxybenzyl)azétidin-3-yloxyl-[3,4']-bipyridinyl-2'-carbonitrile ;
5-(4-méthanesulfonylphényl)-2-[1-(4-phénoxybenzyl)-azétidin-3-yloxy]pyridine ;
5-méthanesulfonyl-6'-[1-(4-phénoxybenzyl)azétidin-3-yloxy-[2,3']bipyridinyle ;
6'-méthanesulfonyl-6-[1-(4-phénoxybenzyl)azétidin-3-yloxy-[3,3']bipyridinyle ;
6'-fluoro-6-[1-(4-phénoxybenzyl)azétidin-3-yloxy]-[3,3']bipyridinyle ;
5-{4-[1-(2,2-diméthylpropyl)azétidin-3-yloxy]phényl}-2-méthanesulfonylpyridine ;
5-[4-(1-benzylazétidin-3-yloxy)phényl]-2-méthane-sulfonylpyridine ;
2-méthanesulfonyl-5-{4-[1-(6-(4-méthoxyphényl)-pyridin-3-ylméthyl)azétidin-3-yloxy]phényl}pyridine ;
2-méthanesulfonyl-5-{4-[1-(3-phénoxybenzyl)azétidin-3-yloxylphényl]pyridine ;
2-méthanesulfonyl-5-{4-[1-(3-méthoxybenzyl)azétidin-3-yloxy]phényl}pyridine ;
5-{4-[1-(4-benzyloxy-3-méthoxybenzyl)azétidin-3-yl-oxy]phényl}-2-méthanesulfonylpyridine ;
5-{4-[1-(3-benzyloxybenzyl)azétidin-3-yloxy]phényl}-2-méthanesulfonylpyridine ;
2-méthanesulfonyl-5-{4-[1-(3-trifluorométhylbenzyl)-azétidin-3-yloxylphényl}pyridine ;
2-méthanesulfonyl-5-{4-[1-(3-méthylbenzyl)azétidin-3-yloxy]phényl}pyridine ;
4-{3-[4-(6-méthanesulfonylpyridin-3-yl)phénoxy]-azétidin-1-ylméthyl}benzonitrile ;
2-méthanesulfonyl-5-{4-[1-(4-méthoxybenzyl)azétidin-3-yloxy]phényl}pyridine ;
5-{4-[1-(4-benzyloxybenzyl)azétidin-3-yloxy]phényl}-2-méthanesulfonylpyridine ;
2-méthanesulfonyl-5-[4-(1-pyridin-2-ylméthylazétidin-3-yloxy)phényl]pyridine ;
2-méthanesulfonyl-5-[4-(1-pyridin-3-ylméthylazétidin-3-yloxy)phényl]pyridine ;
3-{3-[4-(6-méthanesulfonylpyridin-3-yl)phénoxy]-azétidin-1-ylméthyl}quinoline ;
4-(4-{3-[4-(6-méthanesulfonylpyridin-3-yl)phénoxy]-azétidin-1-ylméthyl}phényl)morpholine ;
5-[4-(1-biphényl-4-ylméthylazétidin-3-yloxy)phényl]-2-méthanesulfonylpyridine ;
5-{4-[1-(4-ïsopropylbenzyl)azêtïdïn-3-yloxy]phënyl}-2-méthanesulfonylpyridine ;
[3-(4-{3-[4-(6-méthanesulfonylpyridin-3-yl)phénoxy] - azétidin-1-ylméthyl}phénoxy)propyl]diméthylamine ;
2-méthanesulfonyl-5-{4-[1-(4-propoxybenzyl)azétidin-3-yloxy]phényl}pyridine ;
2-méthanesulfonyl-5-{4-[1-(tétrahydropyran-4-yl-méthyl)azétidin-3-yloxy]phényl}pyridine ;
2-méthanesulfonyl-5-{4-[1-(4-trifluorométhoxybenzyl)-azétidin-3-yloxy]phényl}pyridine ;
5-{4-[1-(2,3-dihydrobenzofuran-5-ylméthyl)azétidin-3-yloxy]phényl}-2-méthanesulfonylpyridine ;
2-{3-[4-(6-méthanesulfonylpyridin-3-yl)phénoxy]-azétidin-1-ylméthyl}quinoline ;
2-méthanesulfonyl-5-{4-[1-(6-méthoxynaphtalén-2-yl-méthyl)azétidin-3-yloxy]phényl}pyridine ;
5-{4-[1-(4-imidazol-1-ylbenzyl)azétidin-3-yloxy]-phényl}-2-méthanesulfonylpyridine ;
5-{4-[1-(4-fluoro-3-phénoxybenzyl)azétidin-3-yloxy]-phényl}-2-méthanesulfonylpyridine ;
2-méthanesulfonyl-5-{4-[1-(4-pyridin-2-ylbenzyl)-azétidin-3-yloxy]phényl}pyridine ;
2-fluoro-5-{3-[4-(6-méthanesulfonylpyridin-3-yl)-phénoxy]azétidin-1-ylméthyl}benzonitrile ;
2-méthanesulfonyl-5-{4-[1-(6-méthoxypyridin-3-yl-méthyl)azétidin-3-yloxylphényl}pyridine ;
5-(4-{3-[4-(6-méthanesulfonylpyridin-3-yl)phénoxy]-azétidin-1-ylméthyl}phényl)pyrimidine ;
2-méthanesulfonyl-5-{4-[1-(6-phénoxypyridin-3-yl-méthyl)azétidin-3-yloxy]phényl}pyridine ;
5-{4-[1-(3,3-diméthylbutyl)azétidin-3-yloxy]phényl}-2-méthanesulfonylpyridine ; et
5-{3-[4-(6-méthanesulfonylpyridin-3-yl)phénoxy]-azétidin-1-ylméthyl}pyrimidine ;
ou un sel pharmaceutiquement acceptable de l'un quelconque de ceux-ci.

18. Composé selon la revendication 1, comprenant de la 5-{4-[1-(4-isopropylbenzyl)azétidin-3-yloxy]phényl}-2-méthanesulfonylpyridine.

19. Composé selon la revendication 1, comprenant de la 2-méthanesulfonyl-5-{4-[1-(4-trifluorométhoxybenzyl)-azétidin-3-yloxy]phényl}pyridine.

20. Composition pharmaceutique comprenant un composé selon l'une quelconque des revendications 1 à 19 ou un sel pharmaceutiquement acceptable de celui-ci et un véhicule pharmaceutiquement acceptable.

21. Composé selon l'une quelconque des revendications 1 à 19, ou sel pharmaceutiquement acceptable de celui-ci, pour utilisation dans le traitement d'une maladie ou d'un état dans lequel GPR119 joue un rôle.

22. Composé selon l'une quelconque des revendications 1 à 19, ou sel pharmaceutiquement acceptable de celui-ci, pour utilisation dans la régulation de la satiété ou le traitement de l'obésité.

23. Composé selon l'une quelconque des revendications 1 à 19, ou sel pharmaceutiquement acceptable de celui-ci, pour utilisation dans le traitement du diabète.

24. Composé selon l'une quelconque des revendications 1 à 19, ou sel pharmaceutiquement acceptable de celui-ci, pour utilisation dans le traitement du syndrome métabolique (syndrome X), d'une altération de la tolérance au glucose, de l'hyperlipidémie, de l'hypertriglycéridémie, de l'hypercholestérolémie, de niveaux bas de HDL ou de l'hypertension.
